Europäisches Patentamt

European Patent Office

Office européen des brevets

(11) Publication number: **0 377 842 B1**

# EUROPEAN PATENT SPECIFICATION

(45) Date of publication of patent specification: **04.10.95**  (51) Int. Cl.6: **C12N  15/48**, A61K 39/21

(21) Application number: **89122964.3**

(22) Date of filing: **12.12.89**

Divisional application 95100871.3 filed on 12/12/89.

(54) **Prototype FeLV isolates for use in disease models and vaccines.**

(30) Priority: **13.12.88 US 284139**

(43) Date of publication of application:
**18.07.90 Bulletin  90/29**

(45) Publication of the grant of the patent:
**04.10.95 Bulletin  95/40**

(84) Designated Contracting States:
**AT BE CH DE ES FR GB GR IT LI LU NL SE**

(56) References cited:
**EP-A- 0 262 887**

**SCIENCE, vol. 239, 19th February 1988, pages 906-910, J. OVERBAUGH et al.: "Molecular cloning of a feline leukemia virus that induces fatal immunodeficiency disease in cats"**

**JOURNAL OF VIROLOGY, vol. 58, no. 3, June 1986, pages 825-834, American Society for Microbiology; M.A. STEWART et al.: "Nucleotide sequences of a feline leukemia virus subgroup a envelope gene and long terminal repeat and evidence for the recombinational origin of subgroup B viruses"**

(73) Proprietor: **HARVARD UNIVERSITY**
**1350 Massachusetts Avenue**
**Cambridge**
**Massachusetts 02138 (US)**

Proprietor: **COLORADO STATE UNIVERSITY RESEARCH FOUNDATION**
**P.O. Box 483,**
**601 South Howes,**
**First Floor**
**Fort Collins,**
**Colorado 80526 (US)**

(72) Inventor: **Hoover, Edward A.**
**1921 Navajo**
**Ft. Collins**
**Colorado 80525 (US)**
Inventor: **Mullins, James I.**
**5 Gorham**
**Brookline, Mass.02146 (US)**

(74) Representative: **Brown, John David et al**
**FORRESTER & BOEHMERT**
**Franz-Joseph-Strasse 38**
**D-80801 München (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

JOURNAL OF VIROLOGY, vol. 62, no. 3, March 1988, pages 722-731, American Society for Microbiology; P.R. DONAHUE et al.: "Strong sequence conservation among horizontally transmissible, minimally pathogenic feline leukemia viruses"

BLOOD, vol. 70, no. 6, December 1987, pages 1880-1892, Grune & Stratton, Inc.; E.A. HOOVER et al.: "Experimental transmission and pathogenesis of immunodeficiency syndrome in cats"

JAVMA, vol. 199(10), 1991, 1392

Vet. Immunol. Immunopathol., vol 11, 1985, 123

J. Virol., vol. 58, 1986, 825

JAVMA, vol. 199(10), 1991, 1453

JAVMA, vol. 19(10), 1991, 1456

Norden News, vol. 3, 1994 Special Edition

**Description**

The present invention is generally directed toward the derivation of molecular clones of FeLV and their use as vaccines and in disease models.

Background of the Invention

Retroviruses form a large class of enveloped RNA viruses which invade a large number of specific mammalian hosts. They are infectiously transmitted by a variety of mechanisms, are frequently associated with severe diseases, and share common elements among their structures. The retroviruses, consisting of a (+) strand RNA dimer (ssRNA), form long terminal repeats ("LTR") in their proviral DNA intermediates and a genome coding for capsid proteins ("the gag gene"), reverse transcriptase and integrase functions (the "pol" gene), and the membrane envelope gene ("env"). With particular viruses, other open reading frames have been shown to be present coding for proteins having specific functions in addition to the functions of the common genes.

Feline leukemia viruses (FeLV) are exogenous type C retroviruses that are responsible for induction of a diverse series of lymphoreticular diseases of outbred cats including lymphosarcoma, leukemia, aplastic anemia, myelodysplasia, and feline acquired immunodeficiency syndrome (Hardy et al., Cancer Res. 36:582, 1976; Hardy, Feline Leukemia Virus, Hardy, Essex, McCelland, eds. (Elsevier/North Holland, 1980), pp. 3-31; Hoover, Rojko, Olsen, Feline Leukemia, Olsen, ed. (CRC Press, Boca Raton, Fla., 1980), pp. 32-51; Hardy and Essex, Prog. Allergy 37:353, 1986). The genome of FeLV is a 60-70S dimer of single-stranded RNA consisting of a gag gene encoding the capsid proteins, a pol gene encoding the protease, reverse transcriptase and integrase, and an env gene encoding the gp70 and p15E viral envelope proteins. As with other retroviruses, when a susceptible cell is infected with FeLV (in vivo or in vitro), the genome is transcribed into a double-stranded DNA copy which is then carried in the cellular DNA as a provirus organized into 5'-LTR (long terminal repeat)-gag-pol-env-LTR-3' regions. The integrated provirus then serves as the template for production of FeLV RNAs and ultimately infectious virions.

FeLV have been classified into subgroups A, B, and C on the basis of virus interference and neutralization assays, and the distribution of the subgroups within feline populations differs markedly. Subgroup A viruses have been found in all naturally occurring infections examined, either alone or in combination with B and C. Subgroup B, found in approximately 40% of all infections, and subgroup C, found in perhaps 1% of infections, occur as mixed infections of subgroups A and B, A and C, or A, B, and C. Subgroup identity also correlates with the host range of infection in vitro and pathogenicity in cats: FeLV-A isolates are sometimes restricted to growth in feline cells and are minimally pathogenic; FeLV-B isolates grow in heterologous cells such as human and canine fibroblastoid cells and are found at a higher frequency in cats with proliferative disease; and the rare FeLV-C isolates have an extended host range including human, canine, and guinea pig cells and are capable of inducing aplastic anemia.

Peterson et al., Vetinary Immunology and Immunopathology, 11, 123-148, 1986, discloses a vaccine, against FeLV-induced disease in cats, comprising whole, killed FeLV virus of subtype B.

In view of the devastating effect of FeLV-induced disease in domestic cats, the prevention of FeLV infection through vaccination of susceptible animals is a high priority for veterinary researchers. Numerous attempts to produce such a vaccine have been largely unsuccessful and, in fact, the only commercially available vaccine (Leukocell™, Norden Laboratories, Lincoln, Nebraska) has been severely criticized for its questionable efficacy (Pedersen et al., Feline Practice 15:7-20, 1985). The present invention fulfills the need in the art for a suitable FeLV vaccine, and further provides other related advantages, including the definition of a prototype highly infectious FeLV-A challenge virus, a prototype molecularly cloned immunodeficiency inducing FeLV, and a disease model useful for the study of retrovirus-induced immunodeficiency syndrome and leukemia in cats and humans.

Disclosure of Invention

Briefly stated, the present invention discloses molecular clones of feline leukemia virus isolates that encode (a) a prototype highly infectious, minimally pathogenic virus, (b) a variant genome that is replication-defective and associated with a fatal immunodeficiency in cats similar to AIDS (Hoover, Blood 70: 1880-1892, 1987; Overbaugh et al., Science 239:906-910, 1988), and (c) a chimeric genome that is replication-competent and induces the immunodeficiency syndrome described above. More specifically, these molecular clones are used to generate cell lines producing infectious virus which is useful in the preparation of vaccines or the generation of viremia or disease challenge systems.

EP 0 377 842 B1

An isolated DNA sequence encoding the proviral genome of a FeLV-A subtype or a biological derivative thereof is disclosed. For purposes of the present invention, FeLV-A subtype or "biological derivative thereof" includes mutants of an FeLV-A subtype which are at least 92% homologous to a FeLV-A subtype. The disclosed DNA sequence may be derived from proviral molecular clone 61C, which is replication-defective and not capable of inducing viremia in feline species (Felis domestica), or clones 61E or EECC, which are capable of inducing persistent viremia in feline species.

Recombinant plasmids capable of directing the expression of the proviral genome of a FeLV-A subtype or biological derivative thereof, and mammalian cells transfected with such a recombinant plasmid are also disclosed. Suitable mammalian cells include feline cells, such as AH927, CRFK, FCWF, Fc9, feline embryo fibroblasts or primary feline cell cultures, and mink lung cells.

According to the present invention, a method of producing a FeLV-A subtype or biological derivative thereof is disclosed. The method generally comprises: (a) transfecting a mammalian host cell with a recombinant plasmid capable of directing the expression of the proviral genome of a FeLV-A subtype or biological derivative thereof, the plasmid comprising a DNA sequence encoding the proviral genome of a FeLV-A subtype or biological derivative thereof; (b) growing the host cell in an appropriate medium; and (c) separating the FeLV-A subtype or biological derivative thereof from the host cells. Suitable DNA sequences include those derived from clones 61E and EECC. Within an alternative embodiment, the mammalian host cell is cotransfected with a recombinant plasmid as briefly described above, the plasmid comprising a DNA sequence derived from the clone 61C and a DNA sequence encoding a replication-competent proviral genome, such as a DNA sequence derived from the clone 61E. The methods may also include, after the step of separating, purifying the FeLV-A subtype or biological derivative thereof by methods well known in the art.

Within a related aspect of the present invention, a method of producing a FeLV vaccine is disclosed. The method generally comprises: (a) transfecting a mammalian host cell with a recombinant plasmid capable of directing the expression of the proviral genome of a FeLV-A subtype or biological derivative thereof; (b) growing the host cell in an appropriate medium; (c) harvesting the FeLV-A subtype or biological derivative immunogens; and (d) inactivating the FeLV-A subtype or biological derivative thereof. Within an alternative embodiment, the mammalian host cell is cotrasfected as described above. The FeLV-A subtype or biological derivative thereof may be inactivated in a number of ways, including exposure to formalin, betapropriolactone or binary ethyleneimine (BEI) under conditions and for a time sufficient to cause inactivation. The method may also include, after the step of inactivating, concentrating the FeLV-A subtype to achieve an antigenic mass suitable for clinical administration to provide protection in the feline species.

There are also disclosed methods for protecting a feline host from FeLV infection. In one such method, an immunogenically effective amount of a composition comprising an inactivated FeLV-A subtype or biological derivative thereof in combination with a physiologically acceptable carrier or diluent is administered to the feline host. Suitable carriers or diluents include sterile water and phosphate buffered saline. The composition may also include a suitable adjuvant, such as complete or incomplete Freund's adjuvant, RIBI, oil adjuvants, particulate adjuvants such as aluminum hydroxide and aluminum phosphate, or general immune stimulating adjuvants, such as avridine and ama 31-91. In one aspect, the physiologically acceptable carrier or diluent comprises cell-free supernatant derived from FeLV-A subtype-producing cells. In this regard, the physiologically acceptable carrier or diluent may further include whole cell lysates of FeLV-A subtype-producing cells. Within a related method, an immunogenically effective amount of a composition comprising the env gene product of a FeLV-A subtype is administered in combination with a physiologically acceptable carrier or diluent.

As noted above, the present invention discloses vaccines against FeLV-induced disease. In one aspect of the present invention, the vaccine comprises an inactivated FeLV-A subtype, as the sole FeLV subtype, or biological derivative thereof, producible by a method according to the present invention, in combination with a suitable adjuvant. Within preferred embodiments, the FeLV-A subtype or biological derivative thereof is encoded by a DNA sequence derived from clone EECC or clone 61E.

Within another aspect of the present invention, the vaccine comprises an inactivated FeLV-A subtype encoded by a DNA sequence derived from a FeLV clone which is replication competent, such as clone 61E, in combination with an inactivated biological derivative of a FeLV-A subtype encoded by a DNA sequence derived from clone 61C, as the sole FeLV subtype along with a physiologically acceptable carrier or diluent.

Feline hosts exhibiting fatal immunodeficiency disease induced by inoculation with a FeLV-A subtype or biological derivative thereof are disclosed. These feline hosts are useful as disease models for both cats and other mammals, including humans.

These and other aspects of the present invention will become evident upon reference to the following detailed description and attached drawings.

4

## Brief Description of the Drawings

Figure 1 is a depiction of the complete nucleotide sequence of clone 61E. The sequence begins at the 5'LTR and extends to the 3'LTR corresponding to the 8440 nucleotide region shown schematically in Figure 2. Deduced amino acid sequences of the two major open reading frames (ORFs) are designated by a single letter code beneath the DNA sequence. Restriction sites also noted in Figure 2 are shown above the DNA sequence.

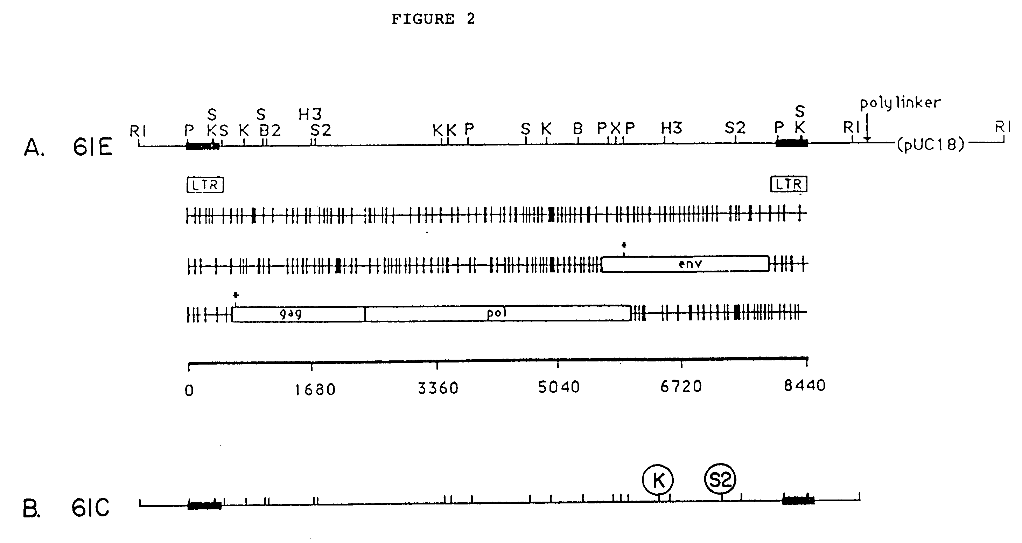

Figure 2A is a restriction site map of clone 61E with location of open reading frames (ORFs). From top to bottom frames 1 to 3 are represented. Vertical bars ( ) mark positions of termination codons. Boxes ( ) represent the ORFs known to encode viral proteins and the positions of the likely initiating methionines for the gag (gag-pol) and env translation products. Restriction sites indicated correspond to BamHI(B), BglII(B2), EcoRI(RI), HindIII(H3) , KpnI(K) , PstI(P) , SmaI(S) , SstII(S2), and XhoI(X). A restriction enzyme map of clone 61C is shown in Figure 2B. Only divergent sites are indicated in the map of the variant clone.

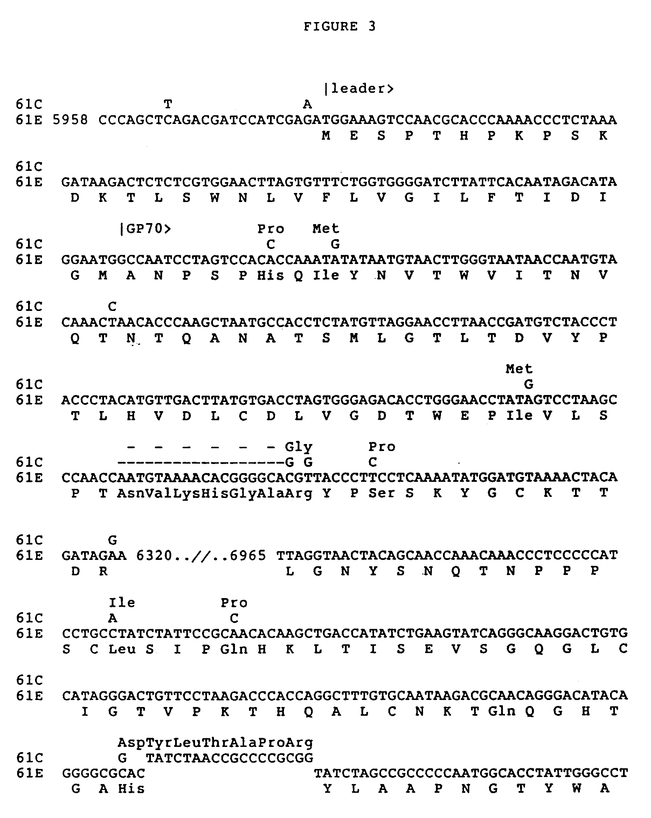

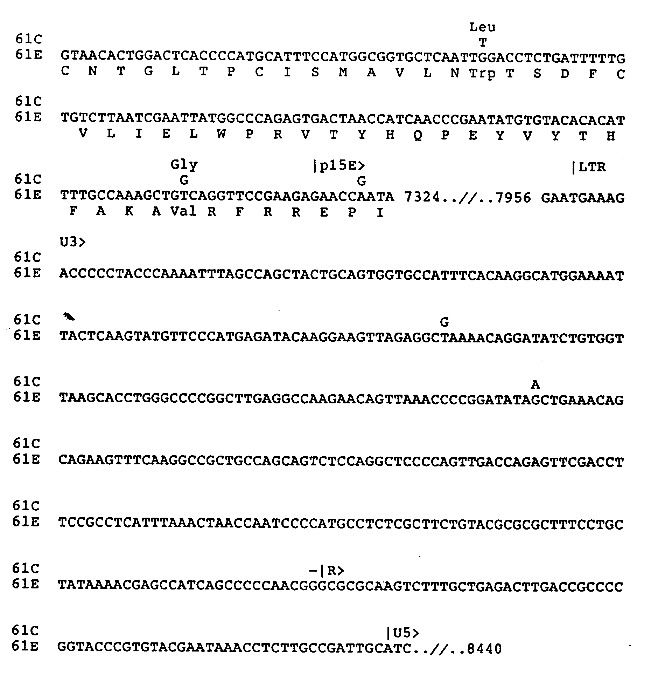

Figure 3 is a depiction of the nucleotide sequence of the env-3'LTR of 61C and comparison to the corresponding region of molecular clone 61E. Portions of the nucleotide sequence of 61E are shown along with the single letter translation of the env protein. The corresponding sequence of clone 61C was determined and only those nucleotide amino acids that differ are shown here. Two stretches of complete nucleotide identity are not shown and are indicated by ../... Where differences result in a change in the deduced amino acid sequence, the three-letter amino acid code is shown. Dashes indicate gaps.

Figure 4a is a depiction of plasmid pUC18-61E.

Figure 4b is a depiction of plasmid pUC18-61C.

Figure 4c is a depiction of plasmid pUC18-EECC.

## Detailed Description of the Invention

As noted above, the subject invention is concerned with methods and compositions for protecting feline hosts from FeLV infection, particularly by providing infectious proviral molecular clones which encode a FeLV of the A subtype or a biological derivative thereof. When certain clones, such as clone 61E, are used to transfect feline cells in vitro, such transfected cells produce infectious virus which is not cytopathic for feline T-lymphocytes or fibroblastoid cells. The growth of this virus is restricted to feline cells (and is therefore ecotropic). Virus derived from these cells is shown herein to infect specific pathogen-free cats from weanling to adult age. None of these cats has developed disease more than one year postinoculation, although they have developed persistent virus infection. Thus, the FeLV-A proviral genome encodes a prototype, ecotropic, horizontally transmissible, and minimally pathogenic feline leukemia virus. It may be distinguished from other feline leukemia viruses, in part, by its ability to consistently induce viremia in cats other than newborn age, thereby mimicking FeLV found in nature more appropriately than any other strain disclosed thus far. The nucleotide sequence of its envelope gene is very highly conserved with other strains of FeLV which are also easily transmitted and found in all FeLV-infected cats in nature. Therefore, an immune response generated to the FeLV-A based vaccines of the present invention is expected to protect against virtually any other feline leukemia virus horizontally transmissible in nature. Furthermore, because of the unique capacity of the virus encoded by DNA sequences derived from clones 61E or EECC to induce a high incidence of viremia in cats inoculated with 61E or EECC, a valid challenge system is provided which proves protection against a homologous virus challenge.

The subject invention is also concerned with methods and compositions for providing a relevant disease model in feline species that may be used to further prove an effective FeLV vaccine (i.e., protection against disease) or that may be used to study possibilities of prophylaxis and therapy of related immunodeficiencies in other species (e.g., human immunodeficiency virus infection in man). The FeLV proviral molecular clone 61C is not infectious, but, when transfected into feline cells, can be rescued by subsequent transfection of molecular clone 61E or infection with 61E virus. The resulting mixture of 61E and 61C virus, when inoculated into 8-week-old susceptible cats, induces a fatal immunodeficiency disease within four months that is typical of that observed in cats inoculated with the original FeLV-FAIDS isolate (Hoover, Blood 70:188-1892, 1987; Overbaugh, Science 239:906-910, 1988). Additionally, a chimeric FeLV proviral molecular clone may be constructed (EECC) by exchanging portions of the 61E and 61C genome. When such a construct is transfected into susceptible feline cells, the resulting virus (FeLV-EECC) is replication competent and induces immunodeficiency disease.

Within preferred embodiments for producing replication competent viruses, FeLV-61E-A and FeLV-EECC provirus are transfected into an appropriate cellular host in culture, for example, the CRFK [ATCC# CCL94] or AH927 cell line (Overbaugh, Science 239:906-910, 1988), conveniently as a provirus plasmid with or without a selectable marker. Methods of transfection may include DEAE dextran precipitation, calcium phosphate precipitation, or electroporation. As a result of the introduction of the proviral DNA, the proviral genome will become integrated into the cellular genome. The transfected cells are selected, expanded and screened for reverse transcriptase, viral production, the level of viral protein antigen released, and any other characteristics associated with the use of the virus as a vaccine.

For producing replication defective viruses, such as FeLV-61C, the proviral genome is transfected into an appropriate cellular host in culture and the resulting transformed line is cotransfected or coinfected with a replication competent proviral genome or virus, such as FeLV-61E.

Stably transfected cell lines which constitutively express FeLV protein and viruses are grown to 100% confluency in 150cm$^2$ roller bottles by seeding, for example, with a minimum of 5-6 X 10$^7$ cells/roller bottle containing 250 ml cell growth medium. After cells have reached confluency in 3-7 days, the cell growth medium is discarded and the cells are replenished, for example, with 250 ml virus growth medium. The cells are further incubated, normally at 37°C, for one to several days and the FeLV containing medium is harvested. Multiple harvest, preferably a minimum of five times, is allowed until the cell monolayer begins to detach from the bottles. The FeLV harvest materials may be concentrated up to 100X depending on virus titer and/or antigenic content, as described for virus fluids. Inactivation, concentration, and adjuvanting of the cell line fluids proceeds as for virus fluids and is described herein.

Once a suitable FeLV-A subtype or biological derivative thereof has been prepared, the feline host may be inoculated by any convenient means with a sufficient amount of the inactivated virus produced from cloned proviral DNA, or with the virus and cell substrate mixtures in order provide an appropriate immune response. The amount of virus utilized will generally be from about 10$^4$ to about 5 x 10$^6$, usually about 1-5 x 10$^5$ focus-forming units/kg host (FFU/kg). The virus may be in any convenient physiologically acceptable medium, e.g., sterile water, phosphate-buffered saline, growth medium or the like. Generally, the dosage volume will be about 0.5 to 2.0 ml, and is administered by injection subcutaneously, intramuscularly, intraperitoneally, intravenously or the like. The vaccines of the present invention may be administered to previously primed hosts. One or more booster injections may be employed at weekly to six-week, usually two- to four-week, intervals.

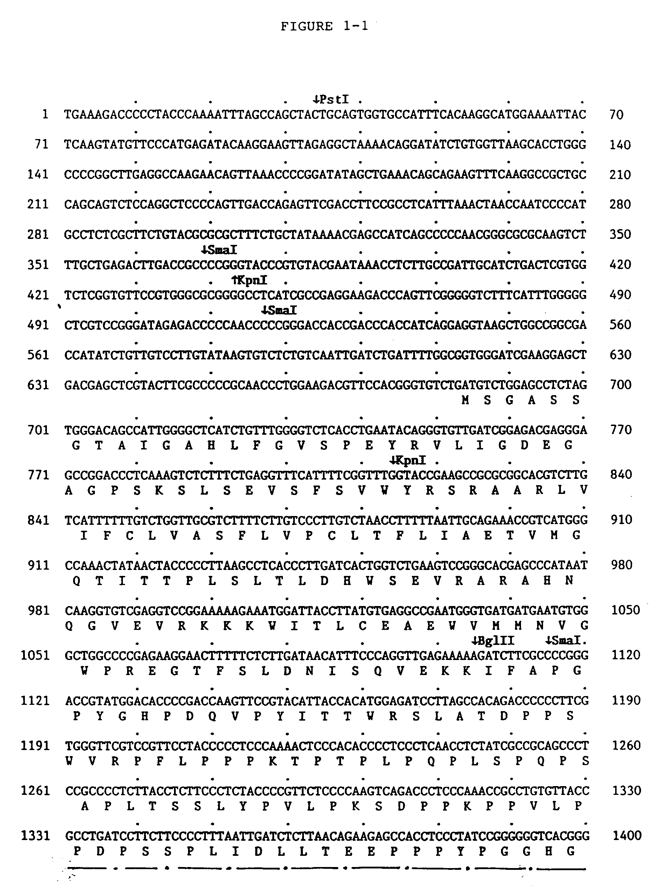

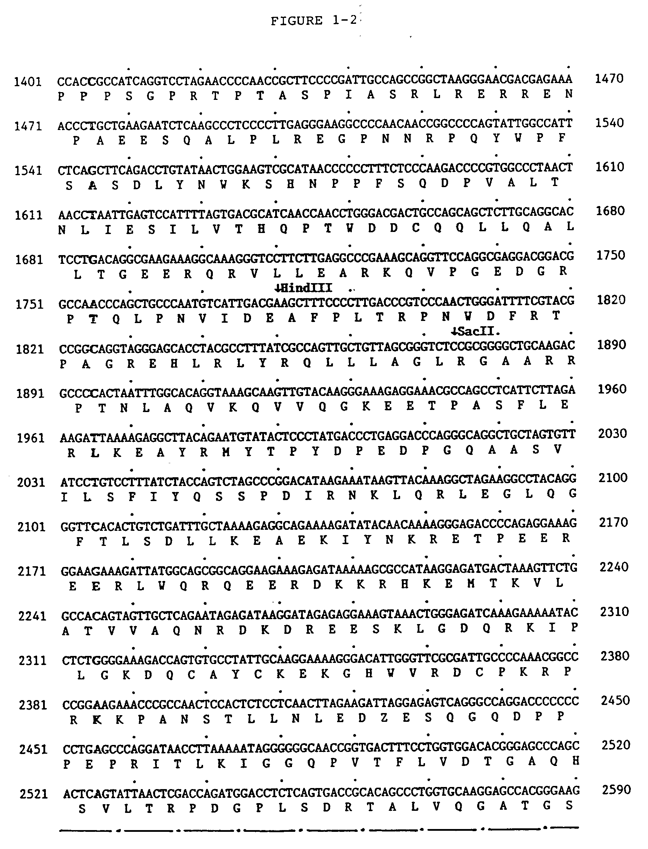

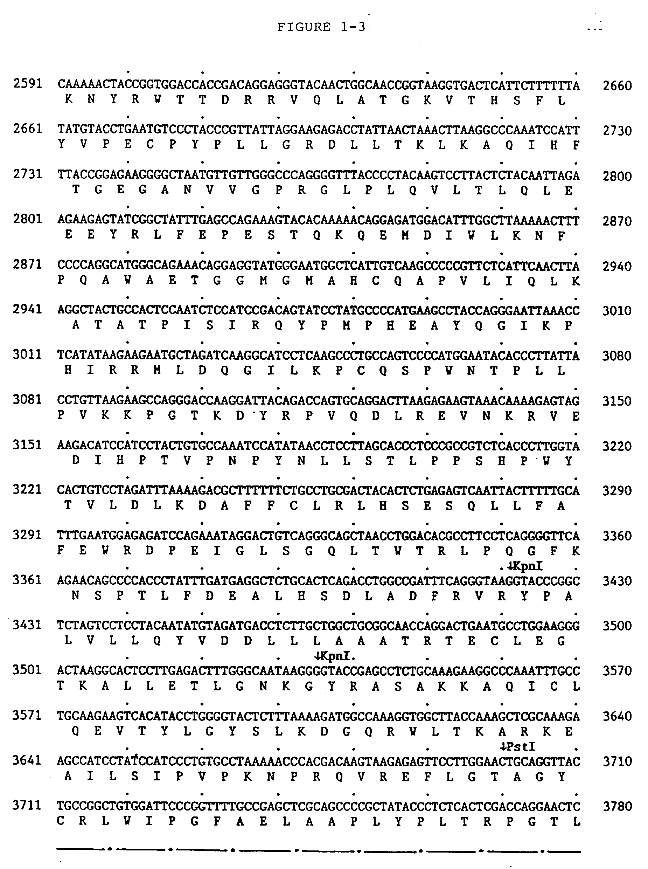

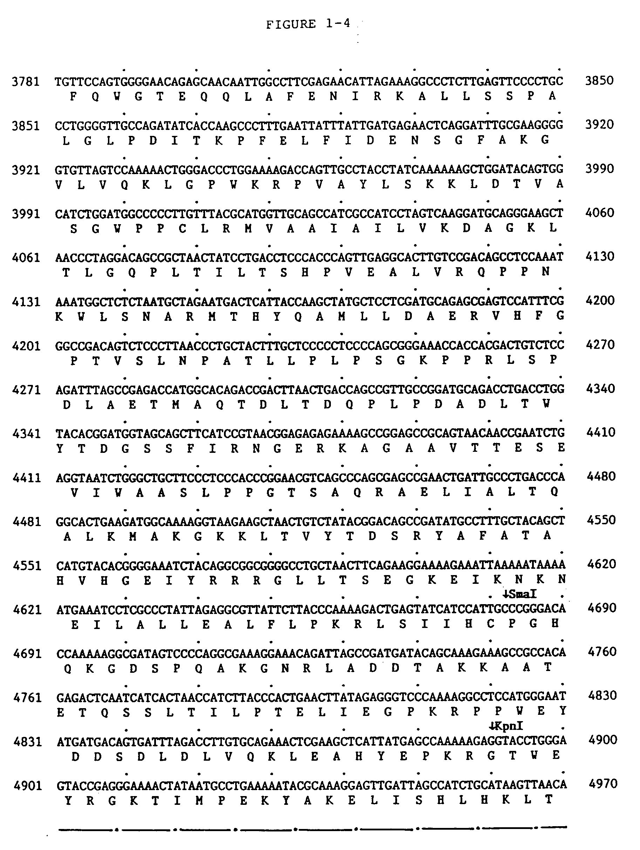

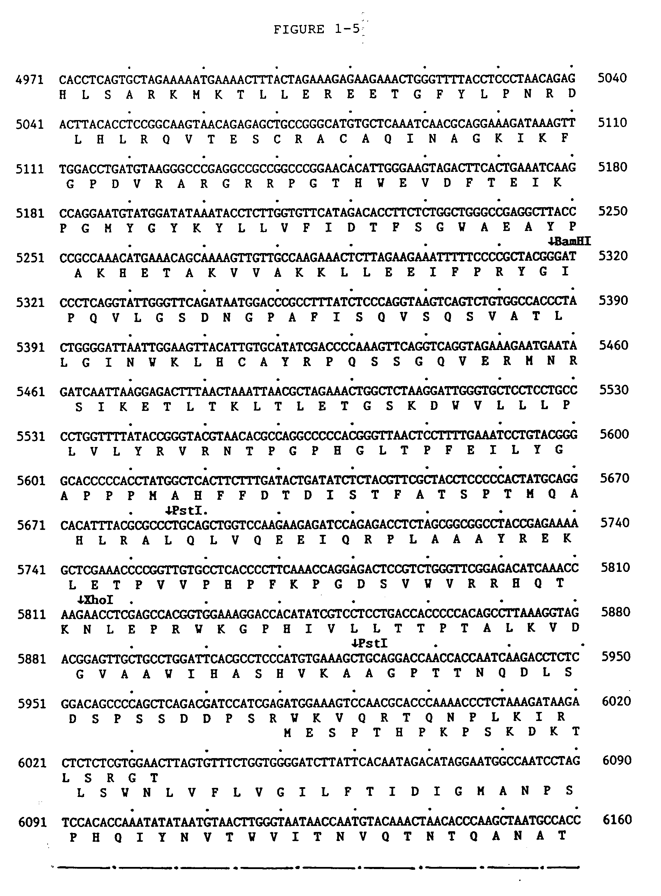

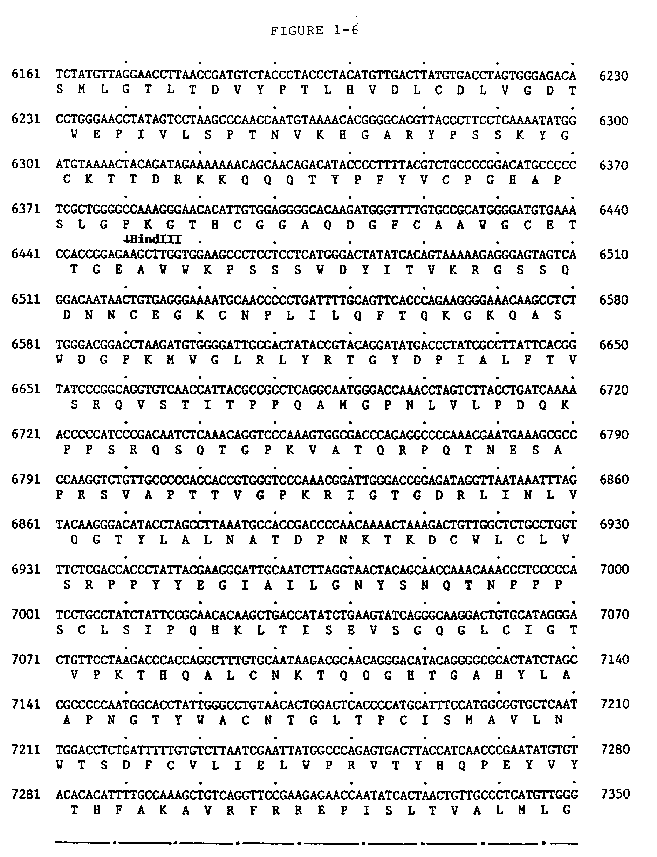

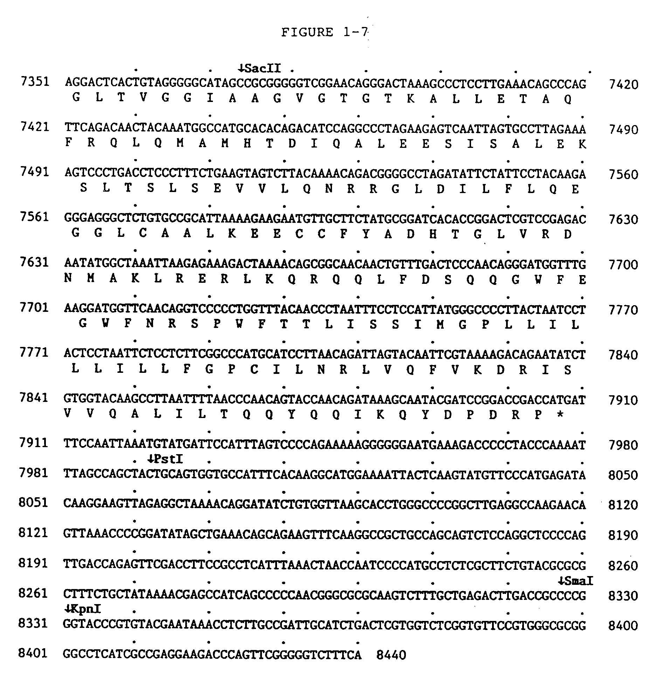

To summarize the examples which follow, the F6A virus-encoding proviral clone 61E was derived directly from intestinal tissue DNA of a specific-pathogen-free cat following inoculation with lymphosarcoma cell-free supernatant from a pet cat with a naturally occurring feline lymphosarcoma from Fort Collins, Colorado. To obtain clones with intact proviruses, DNA from cat 1161 was first cleaved with EcoRI (which does not cleave within the FeLV genome) and fractionated on a sucrose gradient. Fractions containing DNA of sufficient length to potentially contain full-length proviruses, but within the capacity of the bacteriophage vector gtWES B (P. Leder, D. Tiemeier, L. Enquist, Science 196:175, 1977), were pooled. The libraries were prepared and screened with an exogenous LTR-specific probe (Mullins et al., Nature 319:333-336, 1984). An EcoRI fragment of the full-length F6A provirus and host flanking sequences was subcloned into pUC18. Deletion clones were then generated by digestion with exonuclease BAL31, ligated into M13mp18, and sequenced by the dideoxy chain termination method. The complete 8,440 base pair (bp) sequence of the F6A provirus is shown in Figure 1 as well as the deduced amino acid sequences of the two long open reading frames. A simplified restriction map with the location of open reading frames (ORFs) corresponding to gag (encoding the nucleocapsid proteins), pol (encoding protease, reverse transcriptase, and endonucleaseintegrase) and env (encoding the extracellular [gp70] and transmembrane [p15E] envelope proteins) within the F6A sequences is shown in Figure 2. The probable initiating methionines (ATG) of the gag and env genes occur at nucleotide positions 906 and 5981, respectively. In general, the F6A proviral sequences reflect a typical type C retrovirus genome. Particulars of the sequence are discussed in Donahue et a]. (J. Virol. 162:722-731, 1988). Relevant to the present invention, it is significant that when the deduced gp70 protein of F6A is compared to that of two other type A isolates, F3A and FGA (Glasgow), they share remarkably strong (98%) homology, despite their isolation from naturally infected cats up to 13 years apart and from widely separate geographic locations: FGA was isolated in Glasgow, Scotland, in 1970 and carried in culture for several years before being subjected to molecular cloning; F3A was isolated in New York City in 1977 (E. Zuckerman and W.D. Hardy, Jr., personal communication) and was also extensively propagated in culture before being cloned; and F6A was molecularly cloned from cat tissue DNA after one in vivo passage of a virus isolated from a pet cat in 1983 and was never propagated in vitro before being cloned. All three proviruses were molecularly cloned and sequenced. This remarkable sequence conservation may reflect stringent selection against antigenic changes in the ubiquitous, viremia-inducing and horizontally transmissable form of FeLV. This sequence data and comparison, together with the biological activity of F6A described below, show that F6A represents a prototype of the highly

6

conserved, horizontally transmitted, minimally pathogenic subgroup A form of FeLV that is probably present in all naturally infected cats.

To define the biological activity of F6A, a feline embryo fibroblast cell line (AH927) was transfected with cloned 61E DNA. Reverse transcriptase (RT) was detected in the cultures by 12 days, as was proviral DNA. Sixteen 8-week old SPF cats were then inoculated with $10^5$ focus-forming units of 61E-derived F6A virus (titered by clone 81 assay, see below) derived from transfected cells. Each inoculated cat became viremic within two to four weeks and each has remained viremic since then. None has developed any signs of immunodeficiency disease after 10 months to more than 2 years. One cat developed a T⁻ cell lymphosarcoma after 21 months but the others remain healthy, indicating that F6A is minimally pathogenic, although highly infectious, and, like other chronic retroviruses, is capable of inducing lymphosarcoma with a long latency.

The following examples are offered by way of illustration and not by way of limitation.

EXAMPLES

EXAMPLE I

Generation of FeLV Subtype A-Producing Cell Lines by Gene Transfer of a Molecular Clone into Susceptible Feline Cells

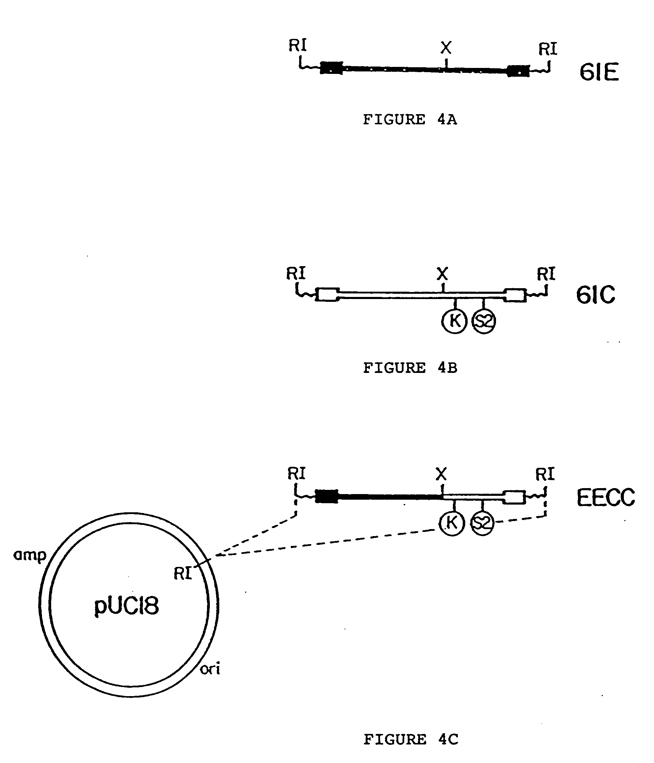

To provide for a vaccine, the FeLV-61E-A provirus was isolated as an EcoRI fragment (Figure 3) and subcloned into the EcoRI site of pUC18 (Figure 4). The resulting plasmid (pUC18-61E) was introduced into the AH927 feline embryo fibroblast cell line by transfection according to the electroporation procedure described by Potter et al. (Proc. Natl. Acad. Sci. (USA) 81:7161-7165, 1984).

The titer of infectious F6A virus released from AH927-61E was found to vary from 4-8 x $10^6$ ffu/ml (Lee et al., J. Natl. Cancer Inst. 49:55-60, 1972).

EXAMPLE II

The following description represents procedures suitable for the inactivation, concentration, and adjuvanting of cell line fluids and virus fluids within the present invention.

Binary Ethyleneimine (BEI) Inactivation of Virus Fluids or Cell Line Fluids

Equal volumes of a 0.2 molar bromoethlamine hydrobromide solution and a 0.4 molar sodium hydroxide solution are mixed and incubated at about 37°C for 60 min. The resulting cyclized inactivant is binary ethyleneimine (BEI) which is added to the virus fluids or cell line fluids at 0.5 to 4%, volume to volume. THe inactivating virus or cell line fluids are held from 4°-37°C for 24 to 72 hr under periodic agitation.

The inactivated virus or cell line fluids are passaged three times in cell culture and examined for specific virus growth to test for complete inactivation.

Concentration of Virus or Cell Culture Fluids

The virus or cell line fluids may be concentrated from 2 to 100 times by any number of available techniques such as Amicon, pellicon (Millipore) concentrating devices, precipitation techniques, such as ammonium chloride or polyethylene glycol (PEG), concentration with Carbowax liquid or wax in conjunction with dialysis tubing, or adjuvant concentration techniques, such as with aluminum phosphate. For the PEG concentration method, 80 ml of 50% PEG is added to 1 liter of virus or cell line fluids, then mixed overnight at 4°C. The next day the PEG-virus fluids are centrifuged at > 2500 RPM, the supernatant is discarded, and the PEG-virus pellet is resuspended in the correct volume of media to achieve the desired concentration.

Adjuvanting Virus or Cell Culture Fluids

The following adjuvants may be used separately or in combination with 2 or more adjuvants depending on interdermal induration reactions in animals and adjuvant mixing compatability.

Ethylene maleic anhydride (EMA) prepared at a 1% weight to volume concentration in water is added to the inactivated virus or cell line fluids at 0.01% to 6% volume to volume (concentration separately or in combination with other adjuvants). The pH of the resulting fluids is adjusted to 7.1 to 7.7 by addition of 1 N sodium hydroxide.

Neocryl A640 is a trade name for a latex emulsion of a copolymer (A or styrene and a mixture of acrylic acid and methacrylic acid). Neocryl A640 is an uncoalesced aqueous acrylic copolymer with styrene, having pH 7.5, viscosity 100 cps (Brookfiled 25°C), weight per gallon is 8.6 lbs as supplied containing 40% solids by weight and 38% solids by volume. The numeral A640 denotes a grade thereof. Other useful Neocryl grades are 520, 625, and 966. The term "CSMA" is used hereinafter to refer to a copolymer of styrene and a mixture of acrylic acid and methacrylic acid. CSMA prepared in a 50% volume per volume suspension in water is added to the inactivated virus or cell line fluids from 0.2 to 10% volume separately or in combination with other adjuvants. Usually there is no need for pH adjustment since the CSMA is a neutral pH.

Modern Veterinary Products (Omaha, Nebr.) Emulsigen adjuvant for small animals is an oil-in-water emulsion which is used separately or in combination with other adjuvants in a 1 to 20% volume to volume of virus or cell line fluids.

Avridine is used separately or in combination with other adjuvants at from 5 to 30 mg per dose. Avridine at 2.4 gm is dissolved in 18 ml of absolute ethyl alcohol, then 1.8 ml of Tween-80 is added and the mixture is passed through a 0.2 micron filter. Subsequently, 20.2 ml of Intralipid soy bean oil is asceptically added to the avridine. Seven to 50% of this adjuvant is then added volume to volume to the virus or cell line fluids.

Raw or purified saponin is used separately or in combination with other adjuvants at from 0.05 mg to 5 mg per dose. Saponin is prepared at a 200 mg/ml concentration, filter sterilized and then added to the virus or cell line fluids at from 0.05 to 20% volume to volume.

Aluminum phosphate at from .01 to 5 mg per dose or aluminum hydroxide at from 0.5 to 20 mg per dose may also be used separately or in combination with other adjuvants.

Cell and Virus Growth Medium

In vaccine production, cells may be grown in minimal essential media (MEM) supplemented with vitamins, nonessential amino acids , sodium pyruvate, sodium bicarbonate (Gibco, Grand Island, NY) containing 10-25 mM Hepes buffer, 30 g/ml polymyxin and 30 g/ml neomycin which has been filter sterilized and stored at 4C. Prior to addition to cells, 2 mM L-glutamine and bovine serum is added to the medium For cell growth, the bovine serum is added to 10%. For cell maintenance, the bovine serum is added to 0.05%. The average harvest is preferably greater than $10^4$ particles/ml.

EXAMPLE III

Use of F6A Virus from the AH927-61E Cell Line as a Vaccine Utilizing Freund's Adjuvant

For purposes of vaccine preparation, AH927-61E cells were grown as follows. F6A virus containing cell Supernatants (2 x $10^5$ ffu/ml) were collected, in some instances concentrated, and inactivated with 0.1%-2% formalin or binary ethylene imine (BEI). Inactivated viral preparations were formulated with complete or incomplete Freund's adjuvant and delivered to SPF cats in 1 ml (?) intramuscular inoculations.

For the studies described in Table 1, at the start of vaccination, cats ranged in age from 4 to 10 months. Two to three doses of vaccine were delivered in the time interval described. Control immunizations included the Norden LEUKOCELL vaccine or adjuvant only. Cats were challenged with either F6A virus from AH927-61E ($10^5$-$10^6$ ffu administered intraperitoneally) or FeLV CSU field isolate virus #05821 ($10^5$ ffu delivered intranasally). The challenge was or was not accompanied by treatment of the cats with corticosteroids as noted in Table 1. Cats were bled from the jugular vein post challenge and assessed for FeLV viremia by p27 ELISA antigen detection in the serum and by immunofluorescence in blood cells. Cats with persistent viremia are considered to have been successfully infected by the challenge. Cats resisting persistent viremia are considered uninfected, i.e., to have resisted challenge. When cats are immunosuppressed, 75%-83% of control animals develop persistent viremia after challenge. Without immunosuppression, 60% of animals develop viremia. In subsequent studies, doses of challenge virus have been increased in order to produce 100% viremia in control animals. Only 5% of all 61E immunized animals developed persistent viremia. In one study (CSUJJ4), 100% of 61E cats resisted viremia from challenge with a heterologous FeLV isolate (05821) of the AB subtype, while only 25% of control cats resisted the challenge. This confirms the ability of the 61E-based vaccine preparation to protect against a heterologous challenge such as would occur in nature. In study CSUFD1, only two inoculations were adequate to protect 100% of animals from viremia (60% of control animals viremic).

TABLE 1

| Vaccine designation | Experiment designation | Vaccine administered on weeks | Total no. doses | Challenge on week no. | Challenge virus | Corticosteroid challenge enhancement used on weeks | Age of cats at start of vaccination (months) | No. cats with persistent viremia[a]/no. cats challenged | Percent of cats which developed persistent viremia (progressors) | Mean percent viremic | Percent of cats which resisted viremia (regressors) | Mean percent resist |
|---|---|---|---|---|---|---|---|---|---|---|---|---|
| FeLV-61E | CSU1 | 0, 2, 4 | 3 | 6 | 05821 | 0, 1 | 6 - 8 | 1 / 6 | 16 | 5 | 84 | 9.5 |
|  | CSUJJ4 | 0, 2, 14 | 3 | 18 | 05821 | 0, 1 | 8 - 9 | 0 / 4 | 0 |  | 100 |  |
|  | CSUFD1 | 0, 2 | 2 | 4 | 61E | none | 7 - 8 | 0 /10  1 /20 | 0 |  | 100 |  |
| Norden LeukocellTM | CSU1 | 0, 2, 4 | 3 | 16 | 05821 | 0, 1 | 4 - 5 | 6 / 6 | 100 | 72.2 | 0 | 27.8 |
|  | CSUJJ4 | 0, 2, 14 | 3 | 18 | 05821 | 0, 1 | 9 - 10 | 1 / 4 | 25 |  | 75 |  |
|  | CSUJJ2 | 0, 2, 10 | 3 | 21 | 05821 | 0, 1 | 6 - 7 | 3 / 4 | 75 |  | 25 |  |
|  | CSUJJ1 | 0, 3, 7, 12 | 4 | 13 | 05821 | 0, 1 | 6 - 8 | 3 / 4  13/18 | 75 |  | 25 |  |
| Adjuvant controls | CSUJJ4 | 0, 2, 14 | 3 | 18 | 05821 | 0, 1 | 6 - 8 | 3 / 4 | 75 | 73.6 | 25 | 26.4 |
|  | CSUJJ3 | 0, 2, 10 | 3 | 16 | 05821 | 0, 1 | 6 - 7 | 3 / 4 | 75 |  | 25 |  |
|  | CSU1 | 0, 2, 4 | 3 | 6 | 05821 | 0, 1 | 8 - 9 | 5 / 6 | 83 |  | 17 |  |
|  | CSUFD1 | 0, 2 | 2 | 4 | 61E | none | 7 - 8 | 3 / 5  14/19 | 60 |  | 40 |  |

[a] - Consistently positive for FeLV gag (p27) antigen in plasma by ELISA and in blood cells by immunofluorescence.

EP 0 377 842 B1

## TABLE 1

| Vaccine designation | Experiment designation | Vaccine administered on weeks | Total no. doses | Challenge on week no. | Challenge virus | Corticosteroid challenge enhancement used on weeks | Age of cats at start of vaccination (months) | No. cats with persistent viremia[a]/ no. cats challenged | Percent of cats which developed persistent viremia (progressors) | Mean percent viremic | Percent of cats which resisted viremia (regressors) | Me pe re |
|---|---|---|---|---|---|---|---|---|---|---|---|---|
| FeLV-61E | CSU1 | 0, 2, 4 | 3 | 6 | 05821 | 0, 1 | 6 - 8 | 1 / 6 | 16 | 5 | 84 | 9. |
| | CSUJJ4 | 0, 2, 14 | 3 | 18 | 05821 | 0, 1 | 8 - 9 | 0 / 4 | 0 | | 100 | |
| | CSUFD1 | 0, 2 | 2 | 4 | 61E | none | 7 - 8 | 0 /10 | 0 | | 100 | |
| | | | | | | | | 1 /20 | | | | |
| Norden Leukocell[TM] | CSU1 | 0, 2, 4 | 3 | 16 | 05821 | 0, 1 | 4 - 5 | 6 / 6 | 100 | 72.2 | 0 | 27 |
| | CSUJJ4 | 0, 2, 14 | 3 | 18 | 05821 | 0, 1 | 9 - 10 | 1 / 4 | 25 | | 75 | |
| | CSUJJ2 | 0, 2, 10 | 3 | 21 | 05821 | 0, 1 | 6 - 7 | 3 / 4 | 75 | | 25 | |
| | CSUJJ1 | 0, 3, 7, 12 | 4 | 13 | 05821 | 0, 1 | 6 - 8 | 3 / 4 | 75 | | 25 | |
| | | | | | | | | 13/18 | | | | |
| Adjuvant controls | CSUJJ4 | 0, 2, 14 | 3 | 18 | 05821 | 0, 1 | 6 - 8 | 3 / 4 | 75 | 73.6 | 25 | 26 |
| | CSUJJ3 | 0, 2, 10 | 3 | 16 | 05821 | 0, 1 | 6 - 7 | 3 / 4 | 75 | | 25 | |
| | CSU1 | 0, 2, 4 | 3 | 6 | 05821 | 0, 1 | 8 - 9 | 5 / 6 | 83 | | 17 | |
| | CSUFD1 | 0, 2 | 2 | 4 | 61E | none | 7 - 8 | 3 / 5 | 60 | | 40 | |
| | | | | | | | | 14/19 | | | | |

a = Consistently positive for FeLV gag (p27) antigen in plasma by ELISA and in blood cells by immunofluorescence.

EXAMPLE IV

Generation of FeLV-Induced Disease Model

In the intestinal DNA preparation from which molecular clone 61E was isolated, an equal copy number of variant genomes (as defined by restriction enzyme polymorphism; see Figure 2) was noted. A molecular clone corresponding to a prototype of the "variant A" genome referred to as 61C was isolated in a bacteriophage vector and subsequently inserted in a plasmid vector (Figure 4b). The plasmid was used to transfect feline fibroblast and T-lymphocyte cultures in vitro. The sequence failed to encode infectious virus. When cotransfected with pFeLV-61E, FeLV-61C was highly infectious and the resulting mixture was cytopathic for T-lymphocytes.

A chimeric virus was constructed in vitro between pFeLV-61E and pFeLV-61C, exchanging sequences on either side of the unique Xho I restriction site found in each provirus at approximately nucleotide position 5817. The 5' terminal sequences were derived from pFeLV-61E, and the 3' sequences were derived from FeLV-61C. The plasmid referred to as pFeLV-EECC (Figure 4c) was used to transfect feline T-lymphocyte cultures in vitro and shown to encode infectious cytopathic virus.

Both the FeLV-61E/61C virus mixture and FeLV-EECC have been shown to induce fatal immunodeficiency disease (Hoover, Blood 70:1880-1892, 1987; Overbaugh, Science 239:906-910, 1988) in all inoculated and persistently viremic cats. The replication-competent FeLV-EECC and similar chimeras induce shorter latency disease, with survival times ranging from 20 to 60 days following inoculation of weanling cats. Survival times for animals inoculated with the FeLV-61E/61C mixture range from 90-120 days.

The nucleotide sequence of the portion of the 61C genome found in the FeLV-EECC chimera was determined and the divergent sequences relative to FeLV-61E identified (Figure 3). The mixture and chimera are the first fully defined retroviruses shown to induce fatal immunodeficiency disease in any organism. They may therefore be used for identification of genetic sequences responsible for AIDS induction in cats, evaluation of anti-viral drug efficacy, as challenge viruses for evaluation of the efficacy of vaccines in preventing viremia and disease, and in the development of vaccines to stimulate immunity against feline leukemia viruses.

Although the foregoing invention has been described in some detail by way of illustration and example for purposes of clarity of understanding, it will be obvious that certain changes and modifications may be practiced within the scope of the appended claims.

The features disclosed in the foregoing description, in the claims and/or in the accompanying drawings may, both, separately and in any combination thereof, be material for realising the invention in diverse forms thereof.

**Claims**

1. A method of producing a FeLV vaccine which method comprises transfecting a mammalian host cell with a recombinant plasmid capable of directing the expression of the proviral genome of a FeLV-A subtype or a biological derivative thereof, said plasmid comprising a DNA sequence encoding the proviral genome of a FeLV-A subtype or a biological derivative thereof, growing said host cell in an appropriate medium, harvesting the FeLV-A subtype or biological derivative immunogens, and inactivating the FeLV-A subtype or biological derivative thereof.

2. A method of producing a FeLV vaccine, which method comprises transfecting a mammalian host cell with a recombinant plasmid capable of directing the expression of the proviral genome of a FeLV-A subtype or a biological derivative thereof, said plasmid comprising a DNA sequence derived from the clone 61C, clone EECC or clone 61E and a DNA sequence derived from a FeLV clone which is replication competent, growing said host cell in an appropriate medium, harvesting the FeLV-A subtype or biological derivative immunogens, and inactivating the FeLV-A subtype or biological derivative thereof.

3. A composition comprising an inactivated FeLV-A subtype as the sole FeLV subtype or a biological derivative thereof, producible by a method according to Claim 1 or 2, in combination with a physiologically acceptable carrier or diluent.

4. A composition according to Claim 3, wherein the composition includes a suitable adjuvant.

5. A vaccine against FeLV-induced disease, comprising an inactivated FeLV-A subtype as the sole FeLV subtype or a biological derivative thereof producible by a method according to Claim 1 or 2, in combination with a suitable adjuvant.

6. A vaccine according to Claim 5, wherein said FeLV-A subtype is encoded by a DNA sequence derived from clone 61E.

7. A vaccine against FeLV-induced disease, which vaccine comprises an inactivated recombinantly produced FeLV-A subtype encoded by a DNA sequence derived from clone 61E in combination with an inactivated FeLV-A subtype encoded by a DNA sequence derived from clone 61C, as the sole FeLV subtype, and a physiologically acceptable carrier of diluent.

8. A vaccine against FeLV-induced disease, which vaccine comprises an inactivated recombinantly produced FeLV-A subtype encoded by a DNA sequence derived from clone 61E, as the sole FeLV subtype, and a physiologically acceptable carrier of diluent.

9. A method of producing a FeLV vaccine, which method comprises co-transfecting a mammalian host cell with a recombinant plasmid capable of directing the expression of the proviral genome of a FeLV-A subtype or a biological derivative thereof, said plasmid comprising a DNA sequence derived from clone 61C, and with a recombinent plasmid capable of directing the expression of the proviral genome of a FeLV-A subtype or a biological derivative thereof, said plasmid comprising a DNA sequence derived from a FeLV clone which is replication competent, growing said host cell in an appropriate medium, harvesting the FeLV-A subtype or biological derivative immunogens, and inactivating the FeLV-A subtype or biological derivative thereof.

**Patentansprüche**

1. Ein Verfahren zur Herstellung eines FeLV-Impfstoffes, wobei das Verfahren Transfektion einer Säugerwirtszelle mit einem rekombinanten Plasmid, das geeignet ist, die Expression des proviralen Genoms eines FeLV-A-Subtyps oder eines biologischen Derivats davon zu leiten, wobei besagtes Plasmid eine DNA-Sequenz umfaßt, die das provirale Genom eines FeLV-A-Subtyps oder eines biologischen Derivats davon codiert, Kultivieren besagter Wirtszelle in einem geeigneten Medium, Ernten der Immunogene des FeLV-A-Subtyps oder biologischen Derivates, und Inaktivieren des FeLV-A-Subtyps oder biologischen Derivats davon umfaßt.

2. Ein Verfahren zur Herstellung eines FeLV-Impfstoffes, wobei das verfahren Transfektion einer Säugerwirtszelle mit einem rekombinanten Plasmid, das geeignet ist, die Expression des proviralen Genoms eines FeLV-A-Subtyps oder eines biologischen Derivats davon zu leiten, wobei besagtes Plasmid eine DNA-Sequenz, die vom Klon 61C, Klon EECC oder Klon 61E abgeleitet ist und eine DNA-Sequenz umfaßt, die von einem FeLV-Klon abgeleitet ist, der replikationskompetent ist, Kultivieren besagter Wirtszelle in einem geeigneten Medium, Ernten der Immunogene des FeLV-A-Subtyps oder biologischen Derivates, und Inaktivieren des FeLV-A-Subtyps, oder biologischen Derivates davon umfaßt.

3. Eine Zusammensetzung, die einen inaktivierten FeLV-A-Subtyp als den einzigen FeLV-Subtyp oder ein biologisches Derivat davon, das durch ein Verfahren nach Anspruch 1 oder 2 herstellbarist,in Kombination mit einem physiologisch akzeptablen Träger oder Verdünnungsmittel umfaßt.

4. Eine Zusammensetzung nach Anspruch 3, wobei die Zusammen-Setzung ein geeignetes Adjuvans einschließt.

5. Ein Impfstoff gegen FeLV-induzierte Krankheit, weicher einen inaktivierten FeLV-A-Subtyp als den einzigen FeLV-Subtyp oder ein biologisches Derivat davon, das durch ein Verfahren nach Anspruch 1 oder 2 herstellbar ist, in Kombination mit einem geeigneten Adjuvans umfaßt.

6. Ein Impfstoff nach Anspruch 5, wobei besagter FeLV-A-Subtyp durch eine DNA-Sequenz codiert ist, die von Klon 61E abgeleitet ist.

**7.** Ein Impfstoff gegen FeLV-induzierte Krankheit, wobei der Impfsoff einen inaktivierten auf rekombinantem Wege produzierten FeLV-A-Subtyp, der durch eine DNA-Sequenz codiert ist, die von Klon 61E abgeleitet ist, in Verbindung mit einem inaktivierten FeLV-A-Subtyp, der durch eine DNA-Sequenz codiert ist, die von Klon 61C, als dem einzige FeLV-Subtyp, abgeleitet ist, und einen physiologisch akzeptablen Träger oder Verdünnungsmittel umfaßt.

**8.** Ein Impfstoff gegen FeLV-induzierte Krankheit, wobei der Impfstoff einen inaktivierten auf rekombinantem Wege hergestellten FeLV-A-Subtyp, der durch eine DNA-Sequenz codiert ist, die von Klon 61E, als dem einzigen FeLV-Untertyp, abgeleitet ist, und einen physiologisch akzeptabler Träger oder Verdünnungsmittel umfaßt.

**9.** Ein Verfahren zur Herstellung eines FeLV-Impfstoffes, wobei das Verfahren Co-Transfektion einer Säugerwirtszelle mit einem rekombinanten Plasmid, das geeignet ist, die Expression des proviralen Genoms eines FeLV-A-Subtyps oder eines biologischen Derivats davon zu leiten, wobei besagtes Plasmid eine DNA-Sequenz umfaßt, die von Klon 61C abgeleitet ist, und mit einem rekombinantem Plasmid, das geeignet ist, die Expression des proviralen Genoms eines FeLV-A-Subtyps oder eines biologischen Derivats davon zu leiten, wobei besagtes Plasmid eine DNA-Sequenz umfaßt, die von einem FeLV-Klon abgeleitet ist, der replikationskompetent ist, Kultivieren besagter Wirtszelle in einem geeigneten Medium, Ernten der Immunogene des FeLV-A-Subtyps oder biologischen Derivates, und Inaktivieren des FeLV-A-Subtyps oder biologischen Derivats davon umfaßt.

**Revendications**

**1.** Procédé de production d,un vaccin contre le FeLV, procédé qui comprend la transfection d'une cellule-hôte de mammifère avec un plasmide recombinant capable de diriger l'expression du génome proviral d'un sous-type FeLV-A ou d'un de ses dérivés biologiques, ledit plasmide comprenant une séquence d'ADN codant pour le génome proviral d'un sous-type FeLV-A ou d'un de ses dérivés biologiques, la culture de ladite cellule-hôte dans un milieu approprié, la collecte d'agents immunognènes du sous-type FeLV-A ou de son dérivé biologique, et l'inactivation du sous-type FeLV-A ou de son dérivé biologique.

**2.** Procédé de production d'un vaccin contre le FeLV, procédé qui comprend la transfection d'une cellule-hôte de mammifère avec un plasmide recombinant capable de diriger l'expression du génome proviral d'un sous-type FeLV-A ou d'un de ses dérivés biologiques, ledit plasmide comprenant une sequence d'ADN dérivée du clone 61C, du clone EECC ou du clone 61E et une séquence d'ADN dérivée d'un clone de FeLV qui est competent du point de vue de la réplication, la culture de ladite cellule-hôte dans un milieu approprié, la collecte d'agents immunogènes du sous-type FeLV-A ou de son dérivé biologique, et l'inactivation du sous-type FeLV-A ou de, son dérivé biologique.

**3.** Composition comprenant un sous-type FeLV-A inactivé comme seul sous-type FeLV, ou un de ses derivés biologiques, pouvant être produit par un procédé suivant la revendication 1 ou 2, en association avec un support ou diluant physiologiquement acceptable.

**4.** Composition suivant la revendication 3, qui comprend un adjuvant convenable.

**5.** Vaccin contre une maladie induite par un FeLV, comprenant un sous-type FeLV-A inactivé comme seul sous-type FeLV, ou un de ses dérivés biologiques, pouvant être produit par un procédé suivant la revendication 1 ou 2, en association avec un adjuvant convenable.

**6.** Vaccin suivant la revendication 5, dans lequel le sous-type FeLV-A est codé par une séquence d'ADN dérivée du clone 61E.

**7.** Vaccin contre une maladie induite par le FeLV, vaccin qui comprend un sous-type FeLV-A produit par recombinaison, inactivé, codé par une séquence d'ADN dérivée du clone 61E en association avec un sous-type FeLV-A inactivé codé par une séquence d'ADN dérivée du clone 61C, comme seul sous-type FeLV, et un support ou diluant physiologiquement acceptable.

**8.** Vaccine contre une maladie induite par le FeLV, vaccin qui comprend un sous-type FeLV-A produit par recombinaison, inactivé, codé par une séquence d'ADN dérivé du clone 61E, comme seul sous-type FeLV, et un support ou diluant physiologiquement acceptable.

**9.** Procédé de production d'un vaccin contre le FeLV, procédé qui comprend la cotransfection d'une cellule-hôte de mammifère avec un plasmide recombinant capable de diriger l'expression du génome proviral d'un sous-type FeLV-A ou d'un de ses dérivés biologiques, ledit plasmide comprenant une séquence d'ADN dérivée du clone 61C, et avec un plasmide recombinant capable de diriger l'expression du génome proviral d'un sous-type FeLV-A ou d'un de ses dérivés biologiques, ledit plasmide comprenant une séquence d'ADN dérivée d'un clone de FeLV qui est compétent du point de vue de la réplication, la culture de ladite cellule-hôte dans un milieu approprié, la collecte des agents immunogènes du sous-type FeLV-A ou de son dérivé biologique, et l'inactivation du sous-type FeLV-A ou de son dérivé biologique.

FIGURE 1-1

```
                                        ↓PstI .       .          .           .
  1  TGAAAGACCCCCTACCCAAAATTTAGCCAGCTACTGCAGTGGTGCCATTTCACAAGGCATGGAAAATTAC   70

 71  TCAAGTATGTTCCCATGAGATACAAGGAAGTTAGAGGCTAAAACAGGATATCTGTGGTTAAGCACCTGGG   140

141  CCCCGGCTTGAGGCCAAGAACAGTTAAACCCCGGATATAGCTGAAACAGCAGAAGTTTCAAGGCCGCTGC   210

211  CAGCAGTCTCCAGGCTCCCCAGTTGACCAGAGTTCGACCTTCCGCCTCATTTAAACTAACCAATCCCCAT   280

281  GCCTCTCGCTTCTGTACGCGCGCTTTCTGCTATAAAACGAGCCATCAGCCCCCAACGGGCGCGCAAGTCT   350
                    ↓SmaI
351  TTGCTGAGACTTGACCGCCCCGGGTACCCGTGTACGAATAAACCTCTTGCCGATTGCATCTGACTCGTGG   420
                     ↑KpnI .        .          .          .
421  TCTCGGTGTTCCGTGGGCGCGGGGCCTCATCGCCGAGGAAGACCCAGTTCGGGGGTCTTTCATTTGGGGG   490
                       ↓SmaI
491  CTCGTCCGGGATAGAGACCCCCAACCCCCGGGACCACCGACCCACCATCAGGAGGTAAGCTGGCCGGCGA   560

561  CCATATCTGTTGTCCTTGTATAAGTGTCTCTGTCAATTGATCTGATTTTGGCGGTGGGATCGAAGGAGCT   630

631  GACGAGCTCGTACTTCGCCCCCGCAACCCTGGAAGACGTTCCACGGGTGTCTGATGTCTGGAGCCTCTAG   700
                                                             M  S  G  A  S  S

701  TGGGACAGCCATTGGGGCTCATCTGTTTGGGGTCTCACCTGAATACAGGGTGTTGATCGGAGACGAGGGA   770
      G  T  A  I  G  A  H  L  F  G  V  S  P  E  Y  R  V  L  I  G  D  E  G
                                                  ↓KpnI .         .         .
771  GCCGGACCCTCAAAGTCTCTTTCTGAGGTTTCATTTTCGGTTTGGTACCGAAGCCGCGCGGCACGTCTTG   840
      A  G  P  S  K  S  L  S  E  V  S  F  S  V  W  Y  R  S  R  A  A  R  L  V

841  TCATTTTTTGTCTGGTTGCGTCTTTTCTTGTCCCTTGTCTAACCTTTTTAATTGCAGAAACCGTCATGGG   910
       I  F  C  L  V  A  S  F  L  V  P  C  L  T  F  L  I  A  E  T  V  M  G

911  CCAAACTATAACTACCCCCTTAAGCCTCACCCTTGATCACTGGTCTGAAGTCCGGGCACGAGCCCATAAT   980
       Q  T  I  T  T  P  L  S  L  T  L  D  H  W  S  E  V  R  A  R  A  H  N

981  CAAGGTGTCGAGGTCCGGAAAAAGAAATGGATTACCTTATGTGAGGCCGAATGGGTGATGATGAATGTGG   1050
       Q  G  V  E  V  R  K  K  K  W  I  T  L  C  E  A  E  W  V  M  M  N  V  G
                                                            ↓BglII      ↓SmaI.
1051 GCTGGCCCCGAGAAGGAACTTTTTTCTCTTGATAACATTTCCCAGGTTGAGAAAAAGATCTTCGCCCCGGG   1120
       W  P  R  E  G  T  F  S  L  D  N  I  S  Q  V  E  K  K  I  F  A  P  G

1121 ACCGTATGGACACCCCGACCAAGTTCCGTACATTACCACATGGAGATCCTTAGCCACAGACCCCCCTTCG   1190
       P  Y  G  H  P  D  Q  V  P  Y  I  T  T  W  R  S  L  A  T  D  P  P  S

1191 TGGGTTCGTCCGTTCCTACCCCCTCCCAAAACTCCCACACCCCTCCCTCAACCTCTATCGCCGCAGCCCT   1260
       W  V  R  P  F  L  P  P  P  K  T  P  T  P  L  P  Q  P  L  S  P  Q  P  S

1261 CCGCCCCTCTTACCTCTTCCCTCTACCCCGTTCTCCCCAAGTCAGACCCTCCCAAACCGCCTGTGTTACC   1330
       A  P  L  T  S  S  L  Y  P  V  L  P  K  S  D  P  P  K  P  P  V  L  P

1331 GCCTGATCCTTCTTCCCCTTTAATTGATCTCTTAACAGAAGAGCCACCTCCCTATCCGGGGGGTCACGGG   1400
       P  D  P  S  S  P  L  I  D  L  L  T  E  E  P  P  P  Y  P  G  G  H  G
```

FIGURE 1-2

```
1401  CCACCGCCATCAGGTCCTAGAACCCCAACCGCTTCCCCGATTGCCAGCCGGCTAAGGGAACGACGAGAAA  1470
      P  P  P  S  G  P  R  T  P  T  A  S  P  I  A  S  R  L  R  E  R  R  E  N

1471  ACCCTGCTGAAGAATCTCAAGCCCTCCCCTTGAGGGAAGGCCCCAACAACCGGCCCCAGTATTGGCCATT  1540
       P  A  E  E  S  Q  A  L  P  L  R  E  G  P  N  N  R  P  Q  Y  W  P  F

1541  CTCAGCTTCAGACCTGTATAACTGGAAGTCGCATAACCCCCCTTTCTCCCAAGACCCCGTGGCCCTAACT  1610
       S  A  S  D  L  Y  N  W  K  S  H  N  P  P  F  S  Q  D  P  V  A  L  T

1611  AACCTAATTGAGTCCATTTTAGTGACGCATCAACCAACCTGGGACGACTGCCAGCAGCTCTTGCAGGCAC  1680
       N  L  I  E  S  I  L  V  T  H  Q  P  T  W  D  D  C  Q  Q  L  L  Q  A  L

1681  TCCTGACAGGCGAAGAAAGGCAAAGGGTCCTTCTTGAGGCCCGAAAGCAGGTTCCAGGCGAGGACGGACG  1750
        L  T  G  E  E  R  Q  R  V  L  L  E  A  R  K  Q  V  P  G  E  D  G  R
                                    ↓HindIII
1751  GCCAACCCAGCTGCCCAATGTCATTGACGAAGCTTTCCCCTTGACCCGTCCCAACTGGGATTTTCGTACG  1820
       P  T  Q  L  P  N  V  I  D  E  A  F  P  L  T  R  P  N  W  D  F  R  T
                                                        ↓SacII.
1821  CCGGCAGGTAGGGAGCACCTACGCCTTTATCGCCAGTTGCTGTTAGCGGGTCTCCGCGGGGCTGCAAGAC  1890
       P  A  G  R  E  H  L  R  L  Y  R  Q  L  L  L  A  G  L  R  G  A  A  R  R

1891  GCCCCACTAATTTGGCACAGGTAAAGCAAGTTGTACAAGGGAAAGAGGAAACGCCAGCCTCATTCTTAGA  1960
       P  T  N  L  A  Q  V  K  Q  V  V  Q  G  K  E  E  T  P  A  S  F  L  E

1961  AAGATTAAAAGAGGCTTACAGAATGTATACTCCCTATGACCCTGAGGACCCAGGGCAGGCTGCTAGTGTT  2030
       R  L  K  E  A  Y  R  M  Y  T  P  Y  D  P  E  D  P  G  Q  A  A  S  V

2031  ATCCTGTCCTTTATCTACCAGTCTAGCCCCGGACATAAGAAATAAGTTACAAAGGCTAGAAGGCCTACAGG  2100
       I  L  S  F  I  Y  Q  S  S  P  D  I  R  N  K  L  Q  R  L  E  G  L  Q  G

2101  GGTTCACACTGTCTGATTTGCTAAAAGAGGCAGAAAAGATATACAACAAAAGGGAGACCCCAGAGGAAAG  2170
        F  T  L  S  D  L  L  K  E  A  E  K  I  Y  N  K  R  E  T  P  E  E  R

2171  GGAAGAAAGATTATGGCAGCGGCAGGAAGAAAGAGATAAAAAGCGCCATAAGGAGATGACTAAAGTTCTG  2240
       E  E  R  L  W  Q  R  Q  E  E  R  D  K  K  R  H  K  E  M  T  K  V  L

2241  GCCACAGTAGTTGCTCAGAATAGAGATAAGGATAGAGAGGAAAGTAAACTGGGAGATCAAAGAAAAATAC  2310
       A  T  V  V  A  Q  N  R  D  K  D  R  E  E  S  K  L  G  D  Q  R  K  I  P

2311  CTCTGGGGAAAGACCAGTGTGCCTATTGCAAGGAAAAGGGACATTGGGTTCGCGATTGCCCCAAACGGCC  2380
        L  G  K  D  Q  C  A  Y  C  K  E  K  G  H  W  V  R  D  C  P  K  R  P

2381  CCGGAAGAAACCCGCCAACTCCACTCTCCTCAACTTAGAAGATTAGGAGAGTCAGGGCCAGGACCCCCCC  2450
       R  K  K  P  A  N  S  T  L  L  N  L  E  D  Z  E  S  Q  G  Q  D  P  P

2451  CCTGAGCCCAGGATAAACCTTAAAAATAGGGGGGCAACCGGTGACTTTCCTGGTGGACACGGGAGCCCAGC  2520
       P  E  P  R  I  T  L  K  I  G  G  Q  P  V  T  F  L  V  D  T  G  A  Q  H

2521  ACTCAGTATTAACTCGACCAGATGGACCTCTCAGTGACCGCACAGCCCTGGTGCAAGGAGCCACGGGAAG  2590
       S  V  L  T  R  P  D  G  P  L  S  D  R  T  A  L  V  Q  G  A  T  G  S
```

16

FIGURE 1-3.

```
2591 CAAAAACTACCGGTGGACCACCGACAGGAGGGTACAACTGGCAACCGGTAAGGTGACTCATTCTTTTTTA 2660
     K  N  Y  R  W  T  T  D  R  R  V  Q  L  A  T  G  K  V  T  H  S  F  L

2661 TATGTACCTGAATGTCCCTACCCGTTATTAGGAAGAGACCTATTAACTAAACTTAAGGCCCAAATCCATT 2730
     Y  V  P  E  C  P  Y  P  L  L  G  R  D  L  L  T  K  L  K  A  Q  I  H  F

2731 TTACCGGAGAAGGGGCTAATGTTGTTGGGCCCAGGGGTTTACCCCTACAAGTCCTTACTCTACAATTAGA 2800
      T  G  E  G  A  N  V  V  G  P  R  G  L  P  L  Q  V  L  T  L  Q  L  E

2801 AGAAGAGTATCGGCTATTTGAGCCAGAAAGTACACAAAAACAGGAGATGGACATTTGGCTTAAAAACTTT 2870
     E  E  Y  R  L  F  E  P  E  S  T  Q  K  Q  E  M  D  I  W  L  K  N  F

2871 CCCCAGGCATGGGCAGAAACAGGAGGTATGGGAATGGCTCATTGTCAAGCCCCCGTTCTCATTCAACTTA 2940
     P  Q  A  W  A  E  T  G  G  M  G  M  A  H  C  Q  A  P  V  L  I  Q  L  K

2941 AGGCTACTGCCACTCCAATCTCCATCCGACAGTATCCTATGCCCCATGAAGCCTACCAGGGAATTAAACC 3010
      A  T  A  T  P  I  S  I  R  Q  Y  P  M  P  H  E  A  Y  Q  G  I  K  P

3011 TCATATAAGAAGAATGCTAGATCAAGGCATCCTCAAGCCCTGCCAGTCCCCATGGAATACACCCTTATTA 3080
      H  I  R  R  M  L  D  Q  G  I  L  K  P  C  Q  S  P  W  N  T  P  L  L

3081 CCTGTTAAGAAGCCAGGGACCAAGGATTACAGACCAGTGCAGGACTTAAGAGAAGTAAACAAAAGAGTAG 3150
     P  V  K  K  P  G  T  K  D  Y  R  P  V  Q  D  L  R  E  V  N  K  R  V  E

3151 AAGACATCCATCCTACTGTGCCAAATCCATATAACCTCCTTAGCACCCTCCCGCCGTCTCACCCTTGGTA 3220
      D  I  H  P  T  V  P  N  P  Y  N  L  L  S  T  L  P  P  S  H  P  W  Y

3221 CACTGTCCTAGATTTAAAAGACGCTTTTTTCTGCCTGCGACTACACTCTGAGAGTCAATTACTTTTTGCA 3290
      T  V  L  D  L  K  D  A  F  F  C  L  R  L  H  S  E  S  Q  L  L  F  A

3291 TTTGAATGGAGAGATCCAGAAATAGGACTGTCAGGGCAGCTAACCTGGACACGCCTTCCTCAGGGGTTCA 3360
     F  E  W  R  D  P  E  I  G  L  S  G  Q  L  T  W  T  R  L  P  Q  G  F  K
                                                          .  ↓KpnI      .
3361 AGAACAGCCCCACCCTATTTGATGAGGCTCTGCACTCAGACCTGGCCGATTTCAGGGTAAGGTACCCGGC 3430
      N  S  P  T  L  F  D  E  A  L  H  S  D  L  A  D  F  R  V  R  Y  P  A

3431 TCTAGTCCTCCTACAATATGTAGATGACCTCTTGCTGGCTGCGGCAACCAGGACTGAATGCCTGGAAGGG 3500
      L  V  L  L  Q  Y  V  D  D  L  L  A  A  A  T  R  T  E  C  L  E  G
                                           ↓KpnI.
3501 ACTAAGGCACTCCTTGAGACTTTGGGCAATAAGGGGTACCGAGCCTCTGCAAAGAAGGCCCAAATTTGCC 3570
     T  K  A  L  L  E  T  L  G  N  K  G  Y  R  A  S  A  K  K  A  Q  I  C  L

3571 TGCAAGAAGTCACATACCTGGGGTACTCTTTAAAAGATGGCCAAAGGTGGCTTACCAAAGCTCGCAAAGA 3640
     Q  E  V  T  Y  L  G  Y  S  L  K  D  G  Q  R  W  L  T  K  A  R  K  E
                                                          ↓PstI       .
3641 AGCCATCCTATCCATCCCTGTGCCTAAAAAACCCACGACAAGTAAGAGAGTTCCTTGGAACTGCAGGTTAC 3710
      A  I  L  S  I  P  V  P  K  N  P  R  Q  V  R  E  F  L  G  T  A  G  Y

3711 TGCCGGCTGTGGATTCCCGGTTTTGCCGAGCTCGCAGCCCCGCTATACCCTCTCACTCGACCAGGAACTC 3780
      C  R  L  W  I  P  G  F  A  E  L  A  A  P  L  Y  P  L  T  R  P  G  T  L
```

17

FIGURE 1-4

3781 TGTTCCAGTGGGGAACAGAGCAACAATTGGCCTTCGAGAACATTAGAAAGGCCCTCTTGAGTTCCCCTGC 3850
     F  Q  W  G  T  E  Q  Q  L  A  F  E  N  I  R  K  A  L  L  S  S  P  A

3851 CCTGGGGTTGCCAGATATCACCAAGCCCTTTGAATTATTTATTGATGAGAACTCAGGATTTGCGAAGGGG 3920
     L  G  L  P  D  I  T  K  P  F  E  L  F  I  D  E  N  S  G  F  A  K  G

3921 GTGTTAGTCCAAAAACTGGGACCCTGGAAAAGACCAGTTGCCTACCTATCAAAAAAGCTGGATACAGTGG 3990
     V  L  V  Q  K  L  G  P  W  K  R  P  V  A  Y  L  S  K  K  L  D  T  V  A

3991 CATCTGGATGGCCCCCTTGTTTACGCATGGTTGCAGCCATCGCCATCCTAGTCAAGGATGCAGGGAAGCT 4060
     S  G  W  P  P  C  L  R  M  V  A  A  I  A  I  L  V  K  D  A  G  K  L

4061 AACCCTAGGACAGCCGCTAACTATCCTGACCTCCCACCCAGTTGAGGCACTTGTCCGACAGCCTCCAAAT 4130
     T  L  G  Q  P  L  T  I  L  T  S  H  P  V  E  A  L  V  R  Q  P  P  N

4131 AAATGGCTCTCTAATGCTAGAATGACTCATTACCAAGCTATGCTCCTCGATGCAGAGCGAGTCCATTTCG 4200
     K  W  L  S  N  A  R  M  T  H  Y  Q  A  M  L  L  D  A  E  R  V  H  F  G

4201 GGCCGACAGTCTCCCTTAACCCTGCTACTTTGCTCCCCCTCCCCAGCGGGAAACCACCACGACTGTCTCC 4270
     P  T  V  S  L  N  P  A  T  L  L  P  L  P  S  G  K  P  P  R  L  S  P

4271 AGATTTAGCCGAGACCATGGCACAGACCGACTTAACTGACCAGCCGTTGCCGGATGCAGACCTGACCTGG 4340
     D  L  A  E  T  M  A  Q  T  D  L  T  D  Q  P  L  P  D  A  D  L  T  W

4341 TACACGGATGGTAGCAGCTTCATCCGTAACGGAGAGAGAAAAGCCGGAGCCGCAGTAACAACCGAATCTG 4410
     Y  T  D  G  S  S  F  I  R  N  G  E  R  K  A  G  A  A  V  T  T  E  S  E

4411 AGGTAATCTGGGCTGCTTCCCTCCCACCCGGAACGTCAGCCCAGCGAGCCGAACTGATTGCCCTGACCCA 4480
     V  I  W  A  A  S  L  P  P  G  T  S  A  Q  R  A  E  L  I  A  L  T  Q

4481 GGCACTGAAGATGGCAAAAGGTAAGAAGCTAACTGTCTATACGGACAGCCGATATGCCTTTGCTACAGCT 4550
     A  L  K  M  A  K  G  K  K  L  T  V  Y  T  D  S  R  Y  A  F  A  T  A

4551 CATGTACACGGGGAAATCTACAGGCGGCGGGGCCTGCTAACTTCAGAAGGAAAAGAAATTAAAAATAAAA 4620
     H  V  H  G  E  I  Y  R  R  R  G  L  L  T  S  E  G  K  E  I  K  N  K  N
                                                              ↓SmaI
4621 ATGAAATCCTCGCCCTATTAGAGGCGTTATTCTTACCCAAAAGACTGAGTATCATCCATTGCCCGGGACA 4690
     E  I  L  A  L  L  E  A  L  F  L  P  K  R  L  S  I  I  H  C  P  G  H

4691 CCAAAAAGGCGATAGTCCCCAGGCGAAAGGAAACAGATTAGCCGATGATACAGCAAAGAAAGCCGCCACA 4760
     Q  K  G  D  S  P  Q  A  K  G  N  R  L  A  D  D  T  A  K  K  A  A  T

4761 GAGACTCAATCATCACTAACCATCTTACCCACTGAACTTATAGAGGGTCCCAAAAGGCCTCCATGGGAAT 4830
     E  T  Q  S  S  L  T  I  L  P  T  E  L  I  E  G  P  K  R  P  P  W  E  Y
                                                       ↓KpnI
4831 ATGATGACAGTGATTTAGACCTTGTGCAGAAACTCGAAGCTCATTATGAGCCAAAAAGAGGTACCTGGGA 4900
     D  D  S  D  L  D  L  V  Q  K  L  E  A  H  Y  E  P  K  R  G  T  W  E

4901 GTACCGAGGGAAAACTATAATGCCTGAAAAATACGCAAAGGAGTTGATTAGCCATCTGCATAAGTTAACA 4970
     Y  R  G  K  T  I  M  P  E  K  Y  A  K  E  L  I  S  H  L  H  K  L  T

FIGURE 1-5

```
4971  CACCTCAGTGCTAGAAAAATGAAAACTTTACTAGAAAGAGAAGAAACTGGGTTTTACCTCCCTAACAGAG  5040
      H  L  S  A  R  K  M  K  T  L  L  E  R  E  E  T  G  F  Y  L  P  N  R  D

5041  ACTTACACCTCCGGCAAGTAACAGAGAGCTGCCGGGCATGTGCTCAAATCAACGCAGGAAAGATAAAGTT  5110
       L  H  L  R  Q  V  T  E  S  C  R  A  C  A  Q  I  N  A  G  K  I  K  F

5111  TGGACCTGATGTAAGGGCCCGAGGCCGCCGGCCCGGAACACATTGGGAAGTAGACTTCACTGAAATCAAG  5180
       G  P  D  V  R  A  R  G  R  R  P  G  T  H  W  E  V  D  F  T  E  I  K

5181  CCAGGAATGTATGGATATAAATACCTCTTGGTGTTCATAGACACCTTCTCTGGCTGGGCCGAGGCTTACC  5250
       P  G  M  Y  G  Y  K  Y  L  L  V  F  I  D  T  F  S  G  W  A  E  A  Y  P
                                                                      ↓BamHI
5251  CCGCCAAACATGAAACAGCAAAAGTTGTTGCCAAGAAACTCTTAGAAGAAATTTTTCCCCGCTACGGGAT  5320
       A  K  H  E  T  A  K  V  V  A  K  K  L  L  E  E  I  F  P  R  Y  G  I

5321  CCCTCAGGTATTGGGTTCAGATAATGGACCCGCCTTTATCTCCCAGGTAAGTCAGTCTGTGGCCACCCTA  5390
       P  Q  V  L  G  S  D  N  G  P  A  F  I  S  Q  V  S  Q  S  V  A  T  L

5391  CTGGGGATTAATTGGAAGTTACATTGTGCATATCGACCCCAAAGTTCAGGTCAGGTAGAAAGAATGAATA  5460
       L  G  I  N  W  K  L  H  C  A  Y  R  P  Q  S  S  G  Q  V  E  R  M  N  R

5461  GATCAATTAAGGAGACTTTAACTAAATTAACGCTAGAAACTGGCTCTAAGGATTGGGTGCTCCTCCTGCC  5530
       S  I  K  E  T  L  T  K  L  T  L  E  T  G  S  K  D  W  V  L  L  L  P

5531  CCTGGTTTTATACCGGGTACGTAACACGCCAGGCCCCCACGGGTTAACTCCTTTTGAAATCCTGTACGGG  5600
       L  V  L  Y  R  V  R  N  T  P  G  H  G  L  T  P  F  E  I  L  Y  G

5601  GCACCCCCACCTATGGCTCACTTCTTTGATACTGATATCTCTACGTTCGCTACCTCCCCCACTATGCAGG  5670
       A  P  P  P  M  A  H  F  F  D  T  D  I  S  T  F  A  T  S  P  T  M  Q  A
                         ↓PstI.
5671  CACATTTACGCGCCCTGCAGCTGGTCCAAGAAGAGATCCAGAGACCTCTAGCGGCGGCCTACCGAGAAAA  5740
        H  L  R  A  L  Q  L  V  Q  E  E  I  Q  R  P  L  A  A  A  Y  R  E  K

5741  GCTCGAAACCCCGGTTGTGCCTCACCCCTTCAAACCAGGAGACTCCGTCTGGGTTCGGAGACATCAAACC  5810
       L  E  T  P  V  V  P  H  P  F  K  P  G  D  S  V  W  V  R  R  H  Q  T
      ↓XhoI
5811  AAGAACCTCGAGCCACGGTGGAAAGGACCACATATCGTCCTCCTGACCACCCCCACAGCCTTAAAGGTAG  5880
       K  N  L  E  P  R  W  K  G  P  H  I  V  L  L  T  T  P  T  A  L  K  V  D
                                                ↓PstI
5881  ACGGAGTTGCTGCCTGGATTCACGCCTCCCATGTGAAAGCTGCAGGACCAACCACCAATCAAGACCTCTC  5950
        G  V  A  A  W  I  H  A  S  H  V  K  A  A  G  P  T  T  N  Q  D  L  S

5951  GGACAGCCCCAGCTCAGACGATCCATCGAGATGGAAAGTCCAACGCACCCAAAACCCTCTAAAGATAAGA  6020
        D  S  P  S  S  D  D  P  S  R  W  K  V  Q  R  T  Q  N  P  L  K  I  R
                                 M  E  S  P  T  H  P  K  P  S  K  D  K  T

6021  CTCTCTCGTGGAACTTAGTGTTTCTGGTGGGGATCTTATTCACAATAGACATAGGAATGGCCAATCCTAG  6090
       L  S  R  G  T
        L  S  W  N  L  V  F  L  V  G  I  L  F  T  I  D  I  G  M  A  N  P  S

6091  TCCACACCAAATATATAATGTAACTTGGGTAATAACCAATGTACAAACTAACACCCAAGCTAATGCCACC  6160
       P  H  Q  I  Y  N  V  T  W  V  I  T  N  V  Q  T  N  T  Q  A  N  A  T
```

FIGURE 1-6

```
6161 TCTATGTTAGGAACCTTAACCGATGTCTACCCTACCCTACATGTTGACTTATGTGACCTAGTGGGAGACA 6230
     S  M  L  G  T  L  T  D  V  Y  P  T  L  H  V  D  L  C  D  L  V  G  D  T

6231 CCTGGGAACCTATAGTCCTAAGCCCAACCAATGTAAAACACGGGGCACGTTACCCTTCCTCAAAATATGG 6300
      W  E  P  I  V  L  S  P  T  N  V  K  H  G  A  R  Y  P  S  S  K  Y  G

6301 ATGTAAAACTACAGATAGAAAAAAACAGCAACAGACATACCCCTTTTACGTCTGCCCCGGACATGCCCCC 6370
      C  K  T  T  D  R  K  K  Q  Q  Q  T  Y  P  F  Y  V  C  P  G  H  A  P

6371 TCGCTGGGGCCAAAGGGAACACATTGTGGAGGGGCACAAGATGGGTTTTGTGCCGCATGGGGATGTGAAA 6440
      S  L  G  P  K  G  T  H  C  G  G  A  Q  D  G  F  C  A  A  W  G  C  E  T
            ↓HindIII
6441 CCACCGGAGAAGCTTGGTGGAAGCCCTCCTCCTCATGGGACTATATCACAGTAAAAAGAGGGAGTAGTCA 6510
      T  G  E  A  W  W  K  P  S  S  S  W  D  Y  I  T  V  K  R  G  S  S  Q

6511 GGACAATAACTGTGAGGGAAAATGCAACCCCCTGATTTTGCAGTTCACCCAGAAGGGGAAACAAGCCTCT 6580
      D  N  N  C  E  G  K  C  N  P  L  I  L  Q  F  T  Q  K  G  K  Q  A  S

6581 TGGGACGGACCTAAGATGTGGGGGATTGCGACTATACCGTACAGGATATGACCCTATCGCCTTATTCACGG 6650
      W  D  G  P  K  M  W  G  L  R  L  Y  R  T  G  Y  D  P  I  A  L  F  T  V

6651 TATCCCGGCAGGTGTCAACCATTACGCCGCCTCAGGCAATGGGACCAAACCTAGTCTTACCTGATCAAAA 6720
      S  R  Q  V  S  T  I  T  P  P  Q  A  M  G  P  N  L  V  L  P  D  Q  K

6721 ACCCCCATCCCGACAATCTCAAACAGGTCCCAAAGTGGCGACCCAGAGGCCCCAAACGAATGAAAGCGCC 6790
      P  P  S  R  Q  S  Q  T  G  P  K  V  A  T  Q  R  P  Q  T  N  E  S  A

6791 CCAAGGTCTGTTGCCCCCACCACCGTGGGTCCCAAACGGATTGGGACCGGAGATAGGTTAATAAATTTAG 6860
      P  R  S  V  A  P  T  T  V  G  P  K  R  I  G  T  G  D  R  L  I  N  L  V

6861 TACAAGGGACATACCTAGCCTTAAATGCCACCGACCCCAACAAAACTAAAGACTGTTGGCTCTGCCTGGT 6930
      Q  G  T  Y  L  A  L  N  A  T  D  P  N  K  T  K  D  C  W  L  C  L  V

6931 TTCTCGACCACCCTATTACGAAGGGATTGCAATCTTAGGTAACTACAGCAACCAAACAAACCCTCCCCCA 7000
      S  R  P  P  Y  Y  E  G  I  A  I  L  G  N  Y  S  N  Q  T  N  P  P  P

7001 TCCTGCCTATCTATTCCGCAACACAAGCTGACCATATCTGAAGTATCAGGGCAAGGACTGTGCATAGGGA 7070
      S  C  L  S  I  P  Q  H  K  L  T  I  S  E  V  S  G  Q  G  L  C  I  G  T

7071 CTGTTCCTAAGACCCACCAGGCTTTGTGCAATAAGACGCAACAGGGACATACAGGGGCGCACTATCTAGC 7140
      V  P  K  T  H  Q  A  L  C  N  K  T  Q  Q  G  H  T  G  A  H  Y  L  A

7141 CGCCCCCAATGGCACCTATTGGGCCTGTAACACTGGACTCACCCCATGCATTTCCATGGCGGTGCTCAAT 7210
      A  P  N  G  T  Y  V  A  C  N  T  G  L  T  P  C  I  S  M  A  V  L  N

7211 TGGACCTCTGATTTTTGTGTCTTAATCGAATTATGGCCCAGAGTGACTTACCATCAACCCGAATATGTGT 7280
      W  T  S  D  F  C  V  L  I  E  L  W  P  R  V  T  Y  H  Q  P  E  Y  V  Y

7281 ACACACATTTTGCCAAAGCTGTCAGGTTCCGAAGAGAACCAATATCACTAACTGTTGCCCTCATGTTGGG 7350
      T  H  F  A  K  A  V  R  F  R  R  E  P  I  S  L  T  V  A  L  M  L  G
```

FIGURE 1-7

```
                               ↓SacII  .            .           .           .            .
7351  AGGACTCACTGTAGGGGGCATAGCCGCGGGGGTCGGAACAGGGACTAAAGCCCTCCTTGAAACAGCCCAG   7420
       G  L  T  V  G  G  I  A  A  G  V  G  T  G  T  K  A  L  L  E  T  A  Q

7421  TTCAGACAACTACAAATGGCCATGCACACAGACATCCAGGCCCTAGAAGAGTCAATTAGTGCCTTAGAAA   7490
       F  R  Q  L  Q  M  A  M  H  T  D  I  Q  A  L  E  E  S  I  S  A  L  E  K

7491  AGTCCCTGACCTCCCTTTCTGAAGTAGTCTTACAAAACAGACGGGGCCTAGATATTCTATTCCTACAAGA   7560
        S  L  T  S  L  S  E  V  V  L  Q  N  R  R  G  L  D  I  L  F  L  Q  E

7561  GGGAGGGCTCTGTGCCGCATTAAAAGAAGAATGTTGCTTCTATGCGGATCACACCGGACTCGTCCGAGAC   7630
       G  G  L  C  A  A  L  K  E  E  C  C  F  Y  A  D  H  T  G  L  V  R  D

7631  AATATGGCTAAATTAAGAGAAAGACTAAAACAGCGGCAACAACTGTTTGACTCCCAACAGGGATGGTTTG   7700
       N  M  A  K  L  R  E  R  L  K  Q  R  Q  Q  L  F  D  S  Q  Q  G  W  F  E

7701  AAGGATGGTTCAACAGGTCCCCCTGGTTTACAACCCTAATTTCCTCCATTATGGGCCCCTTACTAATCCT   7770
        G  W  F  N  R  S  P  W  F  T  T  L  I  S  S  I  M  G  P  L  L  I  L

7771  ACTCCTAATTCTCCTCTTCGGCCCATGCATCCTTAACAGATTAGTACAATTCGTAAAAGACAGAATATCT   7840
        L  L  I  L  L  F  G  P  C  I  L  N  R  L  V  Q  F  V  K  D  R  I  S

7841  GTGGTACAAGCCTTAATTTTTAACCCAACAGTACCAACAGATAAAGCAATACGATCCGGACCGACCATGAT   7910
       V  V  Q  A  L  I  L  T  Q  Q  Y  Q  Q  I  K  Q  Y  D  P  D  R  P  *

7911  TTCCAATTAAATGTATGATTCCATTTAGTCCCCAGAAAAAGGGGGGAATGAAAGACCCCCTACCCAAAAT   7980
              .  ↓PstI   .            .           .            .            .
7981  TTAGCCAGCTACTGCAGTGGTGCCATTTCACAAGGCATGGAAAATTACTCAAGTATGTTCCCATGAGATA   8050

8051  CAAGGAAGTTAGAGGCTAAAACAGGATATCTGTGGTTAAGCACCTGGGCCCCGGCTTGAGGCCAAGAACA   8120

8121  GTTAAACCCCGGATATAGCTGAAACAGCAGAAGTTTCAAGGCCGCTGCCAGCAGTCTCCAGGCTCCCCAG   8190

8191  TTGACCAGAGTTCGACCTTCCGCCTCATTTAAAACTAACCAATCCCCATGCCTCTCGCTTCTGTACGCGCG   8260
                                                                    ↓SmaI
8261  CTTTCTGCTATAAAACGAGCCATCAGCCCCCAACGGGCGCGCAAGTCTTTGCTGAGACTTGACCGCCCCG   8330
       ↓KpnI
8331  GGTACCCGTGTACGAATAAACCTCTTGCCGATTGCATCTGACTCGTGGTCTCGGTGTTCCGTGGGCGCGG   8400

8401  GGCCTCATCGCCGAGGAAGACCCAGTTCGGGGGTCTTTCA   8440
```

FIGURE 2

A. 61E

B. 61C

(Figure 2: restriction maps of clones 61E and 61C, showing LTR, gag, pol, and env regions with restriction sites RI, S, P, K, B, H3, S2, X and scale markers 0, 1680, 3360, 5040, 6720, 8440; polylinker (pUC18) and circled sites K, S2.)

22

FIGURE 3

```
                                    |leader>
61C            T                A
61E 5958 CCCAGCTCAGACGATCCATCGAGATGGAAAGTCCAACGCACCCAAAACCCTCTAAA
                                 M   E   S   P   T   H   P   K   P   S   K


61C
61E GATAAGACTCTCTCGTGGAACTTAGTGTTTCTGGTGGGGATCTTATTCACAATAGACATA
     D   K   T   L   S   W   N   L   V   F   L   V   G   I   L   F   T   I   D   I


         |GP70>             Pro   Met
61C                          C     G
61E GGAATGGCCAATCCTAGTCCACACCAAATATATAATGTAACTTGGGTAATAACCAATGTA
     G   M   A   N   P   S   P His Q Ile Y   N   V   T   W   V   I   T   N   V


61C      C
61E CAAACTAACACCCAAGCTAATGCCACCTCTATGTTAGGAACCTTAACCGATGTCTACCCT
     Q   T   N   T   Q   A   N   A   T   S   M   L   G   T   L   T   D   V   Y   P


                                                          Met
61C                                                        G
61E ACCCTACATGTTGACTTATGTGACCTAGTGGGAGACACCTGGGAACCTATAGTCCTAAGC
     T   L   H   V   D   L   C   D   L   V   G   D   T   W   E   P Ile V   L   S


         -   -   -   -   -   - Gly       Pro
61C      --------------------G G         C
61E CCAACCAATGTAAAACACGGGGCACGTTACCCTTCCTCAAAATATGGATGTAAAACTACA
     P   T AsnValLysHisGlyAlaArg Y   P Ser S   K   Y   G   C   K   T   T


61C      G
61E GATAGAA 6320..//..6965 TTAGGTAACTACAGCAACCAAACAAACCCTCCCCCAT
     D   R                    L   G   N   Y   S   N   Q   T   N   P   P   P


         Ile       Pro
61C      A         C
61E CCTGCCTATCTATTCCGCAACACAAGCTGACCATATCTGAAGTATCAGGGCAAGGACTGTG
     S   C Leu S   I   P Gln H   K   L   T   I   S   E   V   S   G   Q   G   L   C


61C
61E CATAGGGACTGTTCCTAAGACCCACCAGGCTTTGTGCAATAAGACGCAACAGGGACATACA
     I   G   T   V   P   K   T   H   Q   A   L   C   N   K   T Gln Q   G   H   T


         AspTyrLeuThrAlaProArg
61C      G   TATCTAACCGCCCCGCGG
61E GGGGCGCAC                    TATCTAGCCGCCCCCAATGGCACCTATTGGGCCT
     G   A His                    Y   L   A   A   P   N   G   T   Y   W   A
```

23

```
                                                         Leu
61C                                                       T
61E   GTAACACTGGACTCACCCCATGCATTTCCATGGCGGTGCTCAATTGGACCTCTGATTTTTG
       C   N   T   G   L   T   P   C   I   S   M   A   V   L   N  Trp T   S   D   F   C


61C
61E   TGTCTTAATCGAATTATGGCCCAGAGTGACTAACCATCAACCCGAATATGTGTACACACAT
        V   L   I   E   L   W   P   R   V   T   Y   H   Q   P   E   Y   V   Y   T   H


                   Gly                   |p15E>                          |LTR
61C                 G                       G
61E   TTTGCCAAAGCTGTCAGGTTCCGAAGAGAACCAATA 7324..//..7956 GAATGAAAG
       F   A   K   A Val R   F   R   R   E   P   I


       U3>
61C
61E   ACCCCCTACCCAAAATTTAGCCAGCTACTGCAGTGGTGCCATTTCACAAGGCATGGAAAAT


61C                                                          G
61E   TACTCAAGTATGTTCCCATGAGATACAAGGAAGTTAGAGGCTAAAACAGGATATCTGTGGT


61C                                                                   A
61E   TAAGCACCTGGGCCCCGGCTTGAGGCCAAGAACAGTTAAACCCCGGATATAGCTGAAACAG


61C
61E   CAGAAGTTTCAAGGCCGCTGCCAGCAGTCTCCAGGCTCCCCAGTTGACCAGAGTTCGACCT


61C
61E   TCCGCCTCATTTAAACTAACCAATCCCCATGCCTCTCGCTTCTGTACGCGCGCTTTCCTGC


61C                                 -|R>
61E   TATAAAACGAGCCATCAGCCCCCAACGGGCGCGCAAGTCTTTGCTGAGACTTGACCGCCCC


61C                                      |U5>
61E   GGTACCCGTGTACGAATAAACCTCTTGCCGATTGCATC..//..8440
```

24

FIGURE 4A

FIGURE 4B

FIGURE 4C